# EUROPEAN PATENT APPLICATION

(11) **EP 4 310 182 A1**
(43) Date of publication of application: **24.01.2024**
(21) Application number: 22382693.4
(22) Date of filing: 19.07.2022
(51) Int. Cl.: C12N 15/10

(54) **PROTECTED DNA AND METHODS FOR THE PRODUCTION THEREOF**

(71) Applicant: 4basebio UK Ltd, Cambridge CB24 5QE (GB); 4basebio, S.L.U., 28049 Cantoblanco (Madrid) (ES)
(72) Inventor: LANCKRIET, Heikki, Cambridge, CB24 5QE (GB); PICHER, Ángel, 28049 Cantoblanco, Madrid (ES); WALKER, Amy, Cambridge, CB24 5QE (GB)
(74) Representative: Boult Wade Tennant LLP

(57) **Abstract**

Protected DNA comprising a single-stranded DNA (ssDNA) cassette is provided. Further provided are uses of the protected DNA, methods for producing protected DNA, products generated in performing such methods (including intermediate and final products), and kits for use in such methods.

## Description

### TECHNICAL FIELD

The present invention relates to protected DNA comprising a single-stranded DNA (ssDNA) cassette and uses thereof. The present invention also relates to methods for producing a protected DNA, products generated in performing such methods (including intermediate and final products), and kits for use in such methods.

### BACKGROUND

DNA is susceptible to degradation by nucleases which are naturally occurring enzymes within organisms and which have a vital role in the regulation of many cellular processes, while also protecting against foreign DNA species. Enzymatic DNA degradation can render gene therapies ineffective and is a substantial consideration when developing gene therapies or DNA vaccines.

Considerable efforts have been made to extend the effective molecular lifetime of nucleic acids by increasing resistance of the nucleic acid molecules to both extracellular and intracellular nucleases.

For linear molecules, one of the proposed solutions includes the use of phosphorothioated nucleotides (i.e. 2'-deoxynucleotides-5'-(a-thio)-triphosphate).

Phosphorothioated nucleotides comprise a sulphur atom instead of a non-bridging oxygen atom. These modified nucleotides show comparable physical and chemical characteristics to corresponding unmodified nucleotides, but are resistant to exonuclease digestion. As such, the incorporation of the phosphorothioate functional group can prolong the half-life of the nucleic acid molecule.

Phosphorothioate modifications have also been used in the context of a linear double-stranded polynucleotide chain (e.g. double-stranded DNA) to cap the ends of the polynucleotide chain to increase resistance to exonuclease digestion (Putney et al. "A DNA fragment with an alpha-phosphorothioate nucleotide at one end is asymmetrically blocked from digestion by exonuclease III and can be replicated in vivo." Proceedings of the National Academy of Sciences 78.12 (1981): 7350-7354). To cap the ends of the polynucleotide chain, the ends are digested with a restriction enzyme and treated with a DNA polymerase and a mixture of deoxyribonucleotide triphosphates (dNTPs), at least one type of which is a phosphorothioated nucleotide complementary to a nucleotide in the overhanging strand. Since DNA polymerases add nucleotides in the 5' to 3' direction, the result of this treatment is a blunt-ended polynucleotide fragment with a phosphorothioated nucleotide located at the 3'-end of each strand (i.e. in "the cap").

In addition, in therapeutic nucleic acids, phosphorothioated nucleotides are incorporated into short, single-stranded polynucleotide chains. For example, an antisense oligonucleotide fomivirsen is a 21-mer phosphorothioate oligodeoxynucleotide used to treat cytomegalovirus retinitis (Stein and Castanotto, "FDA-approved oligonucleotide therapies in 2017." Molecular Therapy 25.5 (2017): 1069-1075). Similarly, pegaptanib (brand name Macugen) is a short (27-nucleotides) aptamer with a phosphorothioate 3'-3' deoxythymidine cap used for treating age-related macular degeneration of the retina.

However, chemical synthesis of such single-stranded polynucleotides is limited to the generation of relatively short nucleotides. Thus, chemical synthesis of longer single-stranded polynucleotides (>100 mer) presents a number of challenges. For example, as the DNA length starts to exceed 100 nucleotides, the yield of desired products becomes limited by side reactions and even modest inefficiencies within the stepwise chemical reactions have large effects. Thus, there is a need for efficient methods which are capable of generating long single-stranded DNA molecules particularly longer single-stranded DNA molecules which are stable for *in vivo* applications.

### DESCRIPTION

The invention provides a protected DNA comprising a single-stranded DNA (ssDNA) cassette, wherein the protected DNA comprises a nuclease-resistant nucleotide at the 5' end of the ssDNA cassette or 5' of the ssDNA cassette, and a nuclease-resistant nucleotide at the 3'-end of the ssDNA cassette or 3' of the ssDNA cassette. The nuclease-resistant nucleotides are preferably exonuclease-resistant nucleotides e.g. phosphorothioated nucleotides. The location of exonuclease-resistant nucleotides at the 5' and 3' ends of and/or outside of the ssDNA cassette protects the ssDNA cassette from exonuclease digestion. For example, it provides protection from digestion by exonucleases that cleave the 3'-end nucleotides (e.g. exonuclease III) and exonucleases that cleave the 5'-end nucleotides (e.g. exonuclease VIII).

The enhanced resistance to exonuclease digestion extends the life of the ssDNA cassette in a cell (i.e. the ssDNA cassette has enhanced resistance to intracellular exonucleases) and in a cell-free system (i.e. the ssDNA cassette has enhanced resistance to extracellular exonucleases).

As a result of its protection, the ssDNA cassette of the protected DNA may also have prolonged in vivo expression when compared to a linear double-stranded DNA product that does not contain protected nucleotides.

The protected DNA of the present invention has additional advantageous properties, such as a substantial lack of a bacterial backbone and/or antibiotic resistant genes. Lack of these features is particularly beneficial in the production of a cell delivery system, such as a viral vector or a nanoparticle, for example, for cell therapy. Lack of these features makes the linear double-stranded DNA product of the present invention particularly suitable for use in a pharmaceutical composition.

In addition, the present inventors have discovered a method for efficient introduction of protected nucleotides at both the 3'-end and the 5'-end of a ssDNA cassette to form a protected DNA. The invention also provides a method for producing a protected DNA comprising a single-stranded DNA (ssDNA) cassette, wherein the method comprises: (a) providing a partially protected double-stranded DNA (dsDNA) comprising a first strand and a second strand, wherein the first strand of the partially protected dsDNA comprises a cassette, a nuclease-resistant nucleotide at the 5' end of the cassette or 5' of the cassette, and a nuclease-resistant nucleotide at the 3'-end of the cassette or 3' of the cassette; and (b) digesting the second strand of the partially protected dsDNA with an exonuclease thereby generating the protected DNA.

The partially protected dsDNA may be generated by digesting a precursor dsDNA with an endonuclease and ligating first and second adaptor molecules to the digested precursor dsDNA. The first and second adaptor molecules may comprise exonuclease-resistant nucleotides e.g. phosphorothioated nucleotides.

Importantly, the methods enable the high fidelity production of long and stable ssDNA cassettes that have a wide range of applications and uses, as provided herein.

### 1. Protected DNA

The invention provides a protected DNA comprising a single-stranded DNA (ssDNA) cassette, wherein the protected DNA comprises a nuclease-resistant nucleotide at the 5' end of the ssDNA cassette or 5' of the ssDNA cassette, and a nuclease-resistant nucleotide at the 3'-end of the ssDNA cassette or 3' of the ssDNA cassette.

The protected DNA has enhanced resistance to exonuclease digestion. The enhanced resistance to exonuclease digestion may extend the life of the ssDNA cassette in a cell (i.e. the ssDNA cassette may have enhanced resistance to intracellular exonucleases) and/or the enhanced resistance to exonuclease digestion may extend the life of the ssDNA cassette in a cell-free system (i.e. the ssDNA cassette may have enhanced resistance to extracellular exonucleases). The ssDNA cassette is protected from digestion by exonucleases that cleave the 3'-end nucleotides (e.g. exonuclease I), exonucleases that cleave the 5'-end nucleotides (e.g. exonuclease VIII) and exonucleases that cleave both the 3' and 5' end nucleotides (e.g. exonuclease VII). Thus, the ssDNA cassette may have prolonged *in vivo* expression when compared to DNA that does not comprise nuclease-resistant nucleotides as described herein.

The protected DNA may have additional advantageous properties, such as a substantial lack of a bacterial backbone and/or antibiotic resistant genes. Lack of these features is particularly beneficial in the production of a cell delivery system, such as a viral vector or a nanoparticle, for example, for cell therapy. Lack of these features makes protected DNA of the present invention particularly suitable for use in a pharmaceutical composition.

The protected DNA comprising a single-stranded DNA (ssDNA) cassette may comprise a nuclease-resistant nucleotide at the 5' end of the ssDNA cassette or 5' of the ssDNA cassette, and a nuclease-resistant nucleotides at the 3'-end of the ssDNA cassette or 3' of the ssDNA cassette, wherein the cassette comprises at least 100 nucleotides.

The protected DNA comprising a single-stranded DNA (ssDNA) cassette may comprise at least 3 nuclease-resistant nucleotides at the 5' end of the ssDNA cassette or 5' of the ssDNA cassette, and at least 3 nuclease-resistant nucleotides at the 3'-end of the ssDNA cassette or 3' of the ssDNA cassette, optionally wherein the ssDNA cassette comprises at least 100 nucleotides.

The protected DNA comprising a single-stranded DNA (ssDNA) cassette may comprise at least 5 nuclease-resistant nucleotides at the 5' end of the ssDNA cassette or 5' of the ssDNA cassette, and at least 5 nuclease-resistant nucleotides at the 3'-end of the ssDNA cassette or 3' of the ssDNA cassette, optionally wherein the ssDNA cassette comprises at least 100 nucleotides.

The protected DNA comprising a single-stranded DNA (ssDNA) cassette may comprise x nuclease-resistant nucleotides at the 5' end of the ssDNA cassette or 5' of the ssDNA cassette, and y nuclease-resistant nucleotides at the 3'-end of the ssDNA cassette or 3' of the ssDNA cassette, wherein x is at least 1 and y is at least 1, optionally wherein the ssDNA cassette comprises at least 100 nucleotides.

x and y may be independently selected from at least 1, at least 2, at least 3, at least 4 and at least 5. x and y may be different or the same. Preferably x and y are both at least 3. x and y may both be at least 5. x may be 3 and y be 1 and *vice versa.* x may be 3 and y be 5 and *vice versa.*

"Protected DNA" as used herein refers to the protected DNA comprising a single-stranded DNA (ssDNA) cassette unless specified otherwise.

The protected DNA may be resistant to 3'-end exonuclease digestion (e.g. by exonuclease III) and/or 5'-end exonuclease digestion (e.g. by exonuclease VIII). The protected DNA may be resistant to 3'-end exonuclease digestion (e.g. by exonuclease III, exonuclease T and/or exonuclease VII) and/or 5'-end exonuclease digestion (e.g. by exonuclease VIII, exonuclease VII, RecJf, T7 exonuclease, Lamda exonuclease and/or T5 exonuclease).

The protected DNA comprises nuclease-resistant nucleotides (i.e. protected nucleotides), such as phosphorothioated nucleotides.

As used herein the term "protected nucleotide" or "nuclease-resistant nucleotide" is intended to encompass any type of molecule that provides or enhances resistance to nuclease digestion (especially exonuclease digestion). Although the adaptor molecules are described herein as comprising phosphorothioated nucleotides, the skilled person would appreciate that the adaptor molecules may instead comprise any molecules that provide resistance to exonuclease digestion. For example, the adaptor molecules may comprise nuclease resistant nucleotides i.e. modified nucleotides that provide or increase resistance to nucleases (e.g. exonucleases). The adaptor molecules may comprise a peptide, polypeptide or protein that provides or increases resistance to nucleases (e.g. exonucleases) digestion. The adaptor molecules may comprise 2'-O-methyl nucleotides or 2'-O-methoxyethyl (MOE) nucleotides.

As used herein, the term "phosphorothioated nucleotide" refers to a nucleotide that has an altered phosphate backbone, wherein, the sugar moieties are linked by a phosphorothioate bond. In the phosphate backbone of an oligonucleotide sequence, the phosphorothioate bond contains a sulphur atom as a substitute for a non-bridging oxygen atom. This modification renders the internucleotide linkage resistant to nuclease degradation.

The protected DNA may comprise a double stranded region 5' of the cassette. The protected DNA may comprise a double-stranded region 3' of the cassette. The protected DNA may comprise a double-stranded region 5' of the cassette and a double-stranded region 3' of the cassette. Protected DNA comprising double-stranded regions may provide higher efficiency when used as a repair template (or editing template) for CRISPR-Cas mediated homology directed repair (HDR) than a single-stranded repair template. Such a partially double-stranded DNA molecule may also provide lower toxicity compared to a (fully) double-stranded DNA molecule.

The protected DNA may be partially double-stranded. The protected DNA may comprise a portion that is double-stranded and a portion that is single-stranded (i.e. at least the ssDNA cassette).

The protected DNA may be a single-stranded DNA molecule i.e. the protected DNA may not comprise any double-stranded regions.

The protected DNA may comprise a spacer for example in the cassette and/or between the cassette and the nuclease-resistant nucleotides 3' and 5' of the cassette. The spacer may be at least 10, at least 20, at least 30, at least 40, at least 50, at least 60, at least 70, at least 80, at least 90, at least 100, at least 125, at least 150, at least 175, or at least 200 nucleotides long. The spacer may improve cell transfection yields.

The protected DNA may comprise at least 50, at least 100, at least 150, at least 200, at least 250, at least 500, at least 1000, at least 2000, at least 3000, at least 4000, at least 5000, at least 6000, at least 7000, at least 8000, at least 9000, at least 10,000, at least 11,000, at least 12,000, at least 13,000, at least 14,000, or at least 15,000 nucleotides. Preferably, the protected DNA comprises at least 200 nucleotides.

The ssDNA cassette may comprise at least 25, at least 50, at least 100, at least 150, at least 200, at least 210, at least 220, at least 230, at least 240, at least 250, at least 260, at least 270, at least 280, at least 290, at least 300, at least 325, at least 350, at least 375, at least 400, at least 450, at least 500, at least 600, at least 700, at least 800, at least 900, at least 1000, at least 2000, at least 3000, at least 4000, at least 5000, at least 6000, at least 7000, at least 8000, at least 9000, at least 10,000, at least 11,000, at least 12,000, at least 13,000, at least 14,000, or at least 15,000 nucleotides. Preferably, the ssDNA cassette comprises at least 100 nucleotides.

The ssDNA cassette may comprise at least 100 nucleotides, at least 200 nucleotides, 500 nucleotides, 1000 nucleotides, 5000 nucleotides or 10000 nucleotides.

The ssDNA cassette may be a single cassette. The term "single cassette" as used herein is intended to encompass a product that does not comprise or consist of a plurality of cassettes. That is to say that the protected DNA comprises only a single cassette, which may comprise a single coding sequence of a gene of interest. The single cassette may not comprise or consist of a plurality of tandem repeat sequences, and/or concatemeric DNA. The term "single cassette" as used herein is intended to encompass a single copy of the DNA sequence of interest, for example, a single copy of the coding sequence. Thus, the "single cassette" may not encompass a cassette that comprises or consist of multiple copies of the same DNA sequence linked in series. The single cassette may comprise a collection of genes of interest. For example, the single cassette may comprise the sequence for at least two, three, four, or five genes of interest. The genes of interest may not be the same in a single cassette.

The ssDNA cassette may comprise a coding sequence. The coding sequence may encode a gene of interest, for example a gene encoding a protein. The gene may comprise at least 200, at least 500, at least 1000, least 1500, at least 2000, at least 3000, at least 4000, at least 5000, at least 7500, at least 10,000 nucleotides. Preferably, the gene comprises at least 200 nucleotides.

The ssDNA cassette may comprise at least a portion of a promoter and a coding sequence. The cassette may comprise a promoter and a coding sequence. The ssDNA cassette may comprise a promoter, a coding sequence, a ribosomal binding site and a translational termination sequence. The ssDNA cassette may additionally comprise sequences aiding protein expression, such as a cap-independent translation element. The ssDNA cassette may comprise (or encode) a repair template (or editing template). The repair template (or editing template) may be for use in CRISPR-Cas mediated homology directed repair (HDR). The ssDNA cassette may encode CRISPR guide RNA. The ssDNA cassette may be a mammalian expression cassette. The promoter may be a CMV promoter. The cassette may further comprise an enhancer. The cassette may further comprise a reporter gene, such as an eGFP reporter gene or a luciferase reporter gene. The cassette may further comprise a homopolymeric sequence, such as a polyA, poly C, polyT or polyG sequence. The homopolymeric sequence may be between 3-200 nucleotides in length. The homopolymeric sequence may be used to facilitate purification of the cassette, in which case, the homopolymeric sequence may be between 4-12 nucleotides in length, or between 5-10 nucleotides in length. The homopolymeric sequence may be used to improve mRNA expression, in which case, the homopolymeric sequence may be between 10-200 nucleotides in length, preferably between 80-150 nucleotides in length. The homopolymeric sequence may be at least 10, at least 20, at least 30, at least 40, at least 50, at least 60, at least 70, at least 80, at least 90, at least 100, at least 110, at least 120, at least 130, at least 140, at least 150, at least 160, at least 170, at least 180, at least 190, or at least 200 nucleotides in length. For example, the homopolymeric sequence may comprise a polyA sequence of at least 120 nucleotides.

The protected DNA may not comprise a hairpin, a loop or a stem-loop structure.

The protected DNA may additionally comprise a plurality of protected nucleotides (e.g. phosphorothioated nucleotides) at internal positions. For example, the protected DNA may comprise at least 2, at least 4, at least 6, at least 8, at least 10, at least 12, at least 14, at least 16, at least 18, at least 20, at least 30, at least 40, at least 50, at least 60, at least 70, at least 80, at least 90, at least 100, at least 125, at least 150, at least 175, at least 200, at least 250, at least 300, at least 350, at least 400, at least 450, or at least 500 protected nucleotides (e.g. phosphorothioated nucleotides) at internal positions. Preferably, the protected DNA comprises at least 2 protected nucleotides (e.g. phosphorothioated nucleotides) at internal positions.

The internal positions may not be located between the second and penultimate nucleotide of the protected DNA.

The invention provides a protected DNA obtainable by any of the methods described herein.

Aspects of the invention described above in relation to the protected DNA apply *mutatis mutandis* to all aspects of the invention described herein.

### 2. Partially protected double-stranded DNA

The invention provides a partially protected double-stranded DNA (dsDNA) comprising a first strand and a second strand, wherein the first strand comprises: (i) a cassette; (ii) a nuclease-resistant nucleotide at the 5' end of the cassette or 5' of the cassette; and (iii) a nuclease-resistant nucleotide at the 3'-end of the cassette or 3' of the cassette.

The partially protected double-stranded (dsDNA) may be used to produce the protected DNA. Methods for producing the protected DNA from the partially protected dsDNA are provided herein.

The partially protected double-stranded DNA (dsDNA) may comprise a first strand and a second strand, wherein the first strand comprises: (i) a cassette; (ii) a nuclease-resistant nucleotide at the 5' end of the cassette or 5' of the cassette; and (iii) a nuclease-resistant nucleotides at the 3'-end of the cassette or 3' of the cassette; and wherein the second strand of the dsDNA molecule does not comprise any nuclease-resistant nucleotides.

The partially protected dsDNA may comprise a first strand and a second strand, wherein the first strand comprises: (i) a cassette, (ii) at least 3 nuclease-resistant nucleotides at the 5' end of the cassette or 5' of the cassette; and (iii) at least 3 nuclease-resistant nucleotides at the 3'-end of the cassette or 3' of the cassette. The second strand of the dsDNA molecule may not comprise any nuclease-resistant nucleotides.

The partially protected dsDNA may comprise a first strand and a second strand, wherein the first strand comprises: (i) a cassette, (ii) at least 5 nuclease-resistant nucleotides at the 5' end of the cassette or 5' of the cassette; and (iii) at least 5 nuclease-resistant nucleotides at the 3'-end of the cassette or 3' of the cassette. The second strand of the dsDNA molecule may not comprise any nuclease-resistant nucleotides.

The partially protected dsDNA may comprise a first strand and a second strand, wherein the first strand comprises: (i) a cassette; (ii) x nuclease-resistant nucleotides at the 5' end of the cassette or 5' of the cassette; and (iii) y nuclease-resistant nucleotides at the 3'-end of the cassette or 3' of the cassette; wherein x is at least 1 and y is at least 1. The second strand of the dsDNA molecule may not comprise any nuclease-resistant nucleotides.

x and y may be independently selected from at least 1, at least 2, at least 3, at least 4 and at least 5. x and y may be different or the same. Preferably x and y are both at least 3. x and y may both be at least 5. x may be 3 and y be 1 and *vice versa.* x may be 3 and y be 5 and *vice versa.*

The second strand of the partially protected dsDNA is hybridised to the first strand of the partially protected dsDNA. The second strand of the partially protected dsDNA may comprise a sequence complementary to the cassette of the first strand of the partially protected dsDNA.

The second strand of the partially protected dsDNA may comprise a sequence complementary to the cassette, y' nuclease-resistant nucleotides at the 5' end of the sequence complementary to the cassette or 5' of the sequence complementary to the cassette, and x' nuclease-resistant nucleotides at the 3'-end of the sequence complementary to the cassette or 3' of the sequence complementary to the cassette.

x' and y' may be independently selected from 0, at least 1, at least 2, at least 3, at least 4, at least 5. x' and y' may be the same or different. x' may be 0 and y' may be 3 or *vice versa.* x' may be 0 and y' may be 5 or *vice versa.*

In the methods described herein, an exonuclease may be used to digest the second strand of the partially protected dsDNA. If the exonuclease used to digest the second strand of the partially protected dsDNA is a 3' to 5' exonuclease then y may be greater than x. For example, y may be at least 3 or at least 5 and x may be at least 1. If the exonuclease used to digest the second strand of the partially protected dsDNA is a 5' to 3' exonuclease then x may be greater than y. For example, x may be at least 3 or at least 5 and y may be at least 1.

The second strand of the partially protected dsDNA may not comprise any nuclease-resistant nucleotides. The second strand of the partially protected dsDNA may not be resistant to exonuclease digestion. The second strand of the partially protected dsDNA may be susceptible to exonuclease digestion.

The sequence complementary to the cassette may not comprise any nuclease-resistant nucleotides. The sequence complementary to the cassette may not be resistant to exonuclease digestion. The sequence complementary to the cassette may be susceptible to exonuclease digestion.

The first strand of the partially protected dsDNA may be a sense or a coding strand. The first strand of the partially protected dsDNA may be an antisense or non-coding strand. The second strand of the partially protected dsDNA may be a sense or a coding strand. The second strand of the partially protected dsDNA may be an antisense or non-coding strand.

The second strand of the partially protected dsDNA may not be covalently linked to the first strand of the partially protected dsDNA molecule.

The partially protected dsDNA may comprise a spacer for example in the cassette and/or between the cassette and the nuclease-resistant nucleotides 3' and 5' of the cassette. The spacer may be at least 10, at least 20, at least 30, at least 40, at least 50, at least 60, at least 70, at least 80, at least 90, at least 100, at least 125, at least 150, at least 175, or at least 200 nucleotides long.

The partially protected dsDNA may comprise at least 50, at least 100, at least 250, at least 500, at least 1000, at least 2000, at least 3000, at least 4000, at least 5000, at least 6000, at least 7000, at least 8000, at least 9000, at least 10,000, at least 11,000, at least 12,000, at least 13,000, at least 14,000, or at least 15,000 base pairs. Preferably, the partially protected dsDNA comprises at least 200 base pairs.

The cassette may comprise at least 25, at least 50, at least 100, at least 150, at least 200, at least 210, at least 220, at least 230, at least 240, at least 250, at least 260, at least 270, at least 280, at least 290, at least 300, at least 325, at least 350, at least 375, at least 400, at least 450, at least 500, at least 600, at least 700, at least 800, at least 900, at least 1000, at least 2000, at least 3000, at least 4000, at least 5000, at least 6000, at least 7000, at least 8000, at least 9000, at least 10,000, at least 11,000, at least 12,000, at least 13,000, at least 14,000, or at least 15,000 nucleotides. Preferably, the cassette comprises at least 100 nucleotides.

The cassette may be a single cassette. The term "single cassette" as used herein is intended to encompass a product that does not comprise or consist of a plurality of cassettes. That is to say that the partially protected dsDNA comprises only a single cassette, which may comprise a single coding sequence of a gene of interest. The single cassette may not comprise or consist of a plurality of tandem repeat sequences, and/or concatemeric DNA. The term "single cassette" as used herein is intended to encompass a single copy of the DNA sequence of interest, for example, a single copy of the coding sequence. Thus, the "single cassette" may not encompass a cassette that comprises or consist of multiple copies of the same DNA sequence linked in series. The single cassette may comprise a collection of genes of interest. For example, the single cassette may comprise the sequence for at least two, three, four, or five genes of interest. The genes of interest may not be the same in a single cassette.

The partially protected dsDNA may additionally comprise a plurality of protected nucleotides (e.g. phosphorothioated nucleotides) at internal positions. For example, the partially protected DNA may comprise at least 2, at least 4, at least 6, at least 8, at least 10, at least 12, at least 14, at least 16, at least 18, at least 20, at least 30, at least 40, at least 50, at least 60, at least 70, at least 80, at least 90, at least 100, at least 125, at least 150, at least 175, at least 200, at least 250, at least 300, at least 350, at least 400, at least 450, or at least 500 protected nucleotides (e.g. phosphorothioated nucleotides) at internal positions. Preferably, the partially protected DNA comprises at least 2 protected nucleotides (e.g. phosphorothioated nucleotides) at internal positions.

The internal positions may not be located between the second and penultimate nucleotide of the partially protected DNA.

The invention provides a partially protected dsDNA obtainable by any of the methods described herein.

Aspects of the invention described above in relation to the partially protected dsDNA apply *mutatis mutandis* to all aspects of the invention described herein.

### 3. Methods for producing protected DNA and partially protected DNA

The invention provides a method for producing a protected DNA comprising a single-stranded DNA (ssDNA) cassette, wherein the method comprises:
(a) providing a partially protected double-stranded DNA (dsDNA) comprising a first strand and a second strand, wherein the first strand of the partially protected dsDNA comprises a cassette, a nuclease-resistant nucleotide at the 5' end of the cassette or 5' of the cassette, and a nuclease-resistant nucleotide at the 3'-end of the cassette or 3' of the cassette; and
(b) digesting the second strand of the partially protected dsDNA with an exonuclease thereby generating the protected DNA.

The invention provides a method for producing a protected DNA comprising a single-stranded DNA (ssDNA) cassette, wherein the method comprises:
(a) providing a partially protected double-stranded DNA (dsDNA) comprising a first strand and a second strand, wherein the first strand of the partially protected dsDNA comprises a cassette, at least 3 nuclease-resistant nucleotide at the 5' end of the cassette or 5' of the cassette, and at least 3 nuclease-resistant nucleotides at the 3'-end of the cassette or 3' of the cassette; and
(b) digesting the second strand of the partially protected dsDNA with an exonuclease thereby generating the protected DNA.

The invention provides a method for producing a protected DNA comprising a single-stranded DNA (ssDNA) cassette, wherein the method comprises:
(a) providing a partially protected double-stranded DNA (dsDNA) comprising a first strand and a second strand, wherein the first strand of the partially protected dsDNA comprises a cassette, at least 5 nuclease-resistant nucleotide at the 5' end of the cassette or 5' of the cassette, and at least 5 nuclease-resistant nucleotides at the 3'-end of the cassette or 3' of the cassette; and
(b) digesting the second strand of the partially protected dsDNA with an exonuclease thereby generating the protected DNA.

The invention provides a method for producing a protected DNA comprising a single-stranded DNA (ssDNA) cassette, wherein the method comprises:
(a) providing a partially protected double-stranded DNA (dsDNA) comprising a first strand and a second strand, wherein the first strand of the partially protected dsDNA comprises a cassette, x nuclease-resistant nucleotides at the 5' end of the cassette or 5' of the cassette, and y nuclease-resistant nucleotides at the 3'-end of the cassette or 3' of the cassette, wherein x is at least 1 and y is at least 1; and
(b) digesting the second strand of the partially protected dsDNA with an exonuclease thereby generating the protected DNA.

x and y may be independently selected from at least 1, at least 2, at least 3, at least 4 and at least 5. x and y may be different or the same. x and y may both be at least 3. x and y may both be at least 5. x may be 3 and y be 1 and *vice versa.* x may be 3 and y be 5 and *vice versa.*

The method may enable the efficient production of a large quantity of protected DNA. The method may be a cell-free method. The method may enable the production of a product comprising the protected DNA that is substantively free of bacterial contaminants (e.g. remaining after cell lysis).

The partially protected double-stranded DNA (dsDNA) may be any partially protected dsDNA defined herein.

Digesting the second strand of the partially protected dsDNA with an exonuclease may comprise contacting the partially protected dsDNA with an exonuclease to digest the second strand. In this case, the exonuclease may be an exonuclease which acts on double-stranded DNA (e.g. *E*. *coli* exonuclease III, T7 exonuclease, exonuclease V, exonuclease VIII, T5 exonuclease, lambda exonuclease).

Alternatively, digesting the second strand of the partially protected dsDNA with an exonuclease may comprise (a) denaturing the partially protected dsDNA to separate the first and second strands and (b) contacting the second strand with the exonuclease. The partially protected dsDNA may be denatured by means known in the art such as heat and/or alkali conditions.

Heat may include heating the partially protected dsDNA to a temperature of at least 40°C, at least 50°C, at least 60°C, at least 70°C, at least 80°C, or at least 90°C. Preferably, the partially protected dsDNA is heated to at least 95°C. The heating may be carried out for at least 1, at least 3, at least 5, at least 10, at least 15, at least 30 or at least 60 minutes. Preferably, the heating is carried out for at least 3 minutes. Thus, the heating may be at 95°C for at least 3 minutes.

The denatured partially protected dsDNA may be cooled (e.g. to below 20°C, below 10°C or below 5°C) to prevent annealing of the first and second strands. Preferably, the denatured partially protected dsDNA is cooled to around 4°C.

Alkali conditions may be achieved by adding an alkali reagent (e.g. KOH) that creates a pH above 7, 8, 9, 10, 11, 12, 13 or 14. Preferably, an alkali reagent is added that creates a pH above 10. After denaturation, the pH may be lowered by adding an acidic reagent (e.g. HCI). The pH may be lowered by an amount which provides conditions for the exonuclease to be effective at digesting the second strand.

If the partially protected dsDNA is denatured, the exonuclease may be an exonuclease which acts on single-stranded DNA (e.g. *E. coli* exonuclease I, exonuclease VII, exonuclease T, RecJf). The exonuclease may digest DNA from one end (i.e. 5' to 3' or 3' to 5'), resulting in protected DNA comprising a protected nucleotide at one end. The exonuclease may digest DNA from both ends (i.e. 5' to 3' and 3' to 5'), resulting in protected DNA comprising a protected nucleotide on both ends.

The step of exonuclease digestion may allow for the removal of any unprotected strands, strands lacking adaptors at both ends and/or remaining adaptors.

The partially protected dsDNA may be generated by:
(a) contacting a precursor dsDNA comprising a first strand and a second strand with an endonuclease, wherein the precursor dsDNA comprises on the first strand a cassette, an endonuclease target sequence 5' of the cassette and an endonuclease target sequence 3' of the cassette;
(b) digesting the precursor dsDNA with the endonuclease to generate a digested precursor dsDNA;
(c) contacting the digested precursor dsDNA with a ligase and first and second adaptor molecules, wherein the first adaptor molecule comprises a nuclease resistant nucleotide and the second adaptor molecule comprises a nuclease-resistant nucleotide; and
(d) ligating the first adaptor molecule to a first end of the digested precursor dsDNA and ligating the second adaptor molecule to a second end of the digested precursor dsDNA thereby generating the partially protected dsDNA.

The partially protected dsDNA may be generated by:
(a) contacting a precursor dsDNA comprising a first strand and a second strand with an endonuclease, wherein the precursor dsDNA comprises on the first strand a cassette, an endonuclease target sequence 5' of the cassette and an endonuclease target sequence 3' of the cassette;
(b) digesting the precursor dsDNA with the endonuclease to generate a digested precursor dsDNA;
(c) contacting the digested precursor dsDNA with a ligase and first and second adaptor molecules, wherein the first adaptor molecule comprises at least 3 nuclease resistant nucleotides and the second adaptor molecule comprises at least 3 nuclease-resistant nucleotides; and
(d) ligating the first adaptor molecule to a first end of the digested precursor dsDNA and ligating the second adaptor molecule to a second end of the digested precursor dsDNA thereby generating the partially protected dsDNA.

The partially protected dsDNA may be generated by:
(a) contacting a precursor dsDNA comprising a first strand and a second strand with an endonuclease, wherein the precursor dsDNA comprises on the first strand a cassette, an endonuclease target sequence 5' of the cassette and an endonuclease target sequence 3' of the cassette;
(b) digesting the precursor dsDNA with the endonuclease to generate a digested precursor dsDNA;
(c) contacting the digested precursor dsDNA with a ligase and first and second adaptor molecules, wherein the first adaptor molecule comprises at least 5 nuclease resistant nucleotides and the second adaptor molecule comprises at least 5 nuclease-resistant nucleotides; and
(d) ligating the first adaptor molecule to a first end of the digested precursor dsDNA and ligating the second adaptor molecule to a second end of the digested precursor dsDNA thereby generating the partially protected dsDNA.

The partially protected dsDNA may be generated by:
(a) contacting a precursor dsDNA comprising a first strand and a second strand with an endonuclease, wherein the precursor dsDNA comprises on the first strand a cassette, an endonuclease target sequence 5' of the cassette and an endonuclease target sequence 3' of the cassette;
(b) digesting the precursor dsDNA with the endonuclease to generate a digested precursor dsDNA;
(c) contacting the digested precursor dsDNA with a ligase and first and second adaptor molecules, wherein the first adaptor molecule comprises x nuclease resistant nucleotides and the second adaptor molecule comprises y nuclease-resistant nucleotides and wherein x is at least 1 and y is at least 1; and
(d) ligating the first adaptor molecule to a first end of the digested precursor dsDNA and ligating the second adaptor molecule to a second end of the digested precursor dsDNA thereby generating the partially protected dsDNA.

The method for producing a protected DNA comprising a single-stranded DNA (ssDNA) cassette may comprise:
(a) contacting a precursor dsDNA comprising a first and a second strand with an endonuclease, wherein the precursor dsDNA comprises on the first strand a cassette, an endonuclease target sequence 5' of the cassette and an endonuclease target sequence 3' of the cassette;
(b) digesting the precursor dsDNA with the endonuclease to generate a digested precursor dsDNA;
(c) contacting the digested precursor dsDNA with a ligase and first and second adaptor molecules, wherein the first and second adaptor molecules each comprise a nuclease-resistant nucleotide;
(d) ligating the first adaptor molecule to a first end of the digested precursor dsDNA and ligating the second adaptor molecule to a second end of the digested precursor dsDNA thereby generating a partially protected dsDNA, wherein the partially protected dsDNA comprises a first strand and a second strand, wherein the first strand of the partially protected dsDNA comprises the cassette, a nuclease-resistant nucleotide (from the first adaptor molecule) 5' of the cassette, and a nuclease-resistant nucleotide (from the second adaptor molecule) 3' of the cassette;
(e) contacting the partially protected dsDNA with an exonuclease; and
(f) digesting the second strand of the partially protected dsDNA with the exonuclease thereby generating the protected DNA.

The method for producing a protected DNA comprising a single-stranded DNA (ssDNA) cassette may comprise:
(a) contacting a precursor dsDNA comprising a first and a second strand with an endonuclease, wherein the precursor dsDNA comprises on the first strand a cassette, an endonuclease target sequence 5' of the cassette and an endonuclease target sequence 3' of the cassette;
(b) digesting the precursor dsDNA with the endonuclease to generate a digested precursor dsDNA;
(c) contacting the digested precursor dsDNA with a ligase and first and second adaptor molecules, wherein the first and second adaptor molecules each comprise at least 3 nuclease-resistant nucleotides;
(d) ligating the first adaptor molecule to a first end of the digested precursor dsDNA and ligating the second adaptor molecule to a second end of the digested precursor dsDNA thereby generating a partially protected dsDNA, wherein the partially protected dsDNA comprises a first strand and a second strand, wherein the first strand of the partially protected dsDNA comprises the cassette, at least 3 nuclease-resistant nucleotides (from the first adaptor molecule) 5' of the cassette, and at least 3 nuclease-resistant nucleotides (from the second adaptor molecule) 3' of the cassette;
(e) contacting the partially protected dsDNA with an exonuclease; and
(f) digesting the second strand of the partially protected dsDNA with the exonuclease thereby generating the protected DNA.

The method for producing a protected DNA comprising a single-stranded DNA (ssDNA) cassette may comprise:
(a) contacting a precursor dsDNA comprising a first and a second strand with an endonuclease, wherein the precursor dsDNA comprises on the first strand a cassette, an endonuclease target sequence 5' of the cassette and an endonuclease target sequence 3' of the cassette;
(b) digesting the precursor dsDNA with the endonuclease to generate a digested precursor dsDNA;
(c) contacting the digested precursor dsDNA with a ligase and first and second adaptor molecules, wherein the first and second adaptor molecules each comprise at least 5 nuclease-resistant nucleotides;
(d) ligating the first adaptor molecule to a first end of the digested precursor dsDNA and ligating the second adaptor molecule to a second end of the digested precursor dsDNA thereby generating a partially protected dsDNA, wherein the partially protected dsDNA comprises a first strand and a second strand, wherein the first strand of the partially protected dsDNA comprises the cassette, at least 5 nuclease-resistant nucleotides (from the first adaptor molecule) 5' of the cassette, and at least 5 nuclease-resistant nucleotides (from the second adaptor molecule) 3' of the cassette;
(e) contacting the partially protected dsDNA with an exonuclease; and
(f) digesting the second strand of the partially protected dsDNA with the exonuclease thereby generating the protected DNA.

The method for producing a protected DNA comprising a single-stranded DNA (ssDNA) cassette may comprise:
(a) contacting a precursor dsDNA comprising a first and a second strand with an endonuclease, wherein the precursor dsDNA comprises on the first strand a cassette, an endonuclease target sequence 5' of the cassette and an endonuclease target sequence 3' of the cassette;
(b) digesting the precursor dsDNA with the endonuclease to generate a digested precursor dsDNA;
(c) contacting the digested precursor dsDNA with a ligase and first and second adaptor molecules, wherein the first adaptor molecule comprises x nuclease-resistant nucleotides and the second adaptor molecule comprises y nuclease-resistant nucleotides, and wherein x is at least 1 and y is at least 1;
(d) ligating the first adaptor molecule to a first end of the digested precursor dsDNA and ligating the second adaptor molecule to a second end of the digested precursor dsDNA thereby generating a partially protected dsDNA, wherein the partially protected dsDNA comprises a first strand and a second strand, wherein the first strand of the partially protected dsDNA comprises the cassette, x nuclease-resistant nucleotides (from the first adaptor molecule) 5' of the cassette, and y nuclease-resistant nucleotides (from the second adaptor molecule) 3' of the cassette;
(e) contacting the partially protected dsDNA with an exonuclease; and
(f) digesting the second strand of the partially protected dsDNA with the exonuclease thereby generating the protected DNA.

x and y may be independently selected from at least 1, at least 2, at least 3, at least 4 and at least 5. x and y may be different or the same. Preferably x and y are both at least 3. x and y may both be at least 5. x may be 3 and y be 1 and *vice versa.* x may be 3 and y be 5 and *vice versa.*

Steps (a) (i.e. contacting a precursor dsDNA comprising a first and a second strand with an endonuclease) to (d) (i.e. ligating the first and second adaptor molecules to the digested precursor dsDNA) may be performed in a single contiguous aqueous volume.

The precursor dsDNA may comprise one or more cleavable (e.g. endonuclease) target sequences. The precursor dsDNA may comprise two cleavable (e.g. endonuclease) target sequences. The one or more endonuclease target sequences may be Type IIS endonuclease target sequences. The one or more endonuclease target sequences may be BbsI, BsaI, BsmBI, BspQI, BtgZI, Esp3I,SapI, AarI, Acc36I AcIWI, AcuI, AjuI, AloI, AIw26I, AlwI, ArsI, AsuHPI, BaeI, BarI, BbvI, BccI, BceAI, BcgI, BciVI, BcoDI, BfuAI, BfuI, BmrI, BmsI, BmuI, BpiI, BpmI, BpuEI, BsaXI, Bse1I, Bse3DI, BseGI, BseMI, BseMII, BseNI, BseRI, BseXI, Bsgl, BsIFI, BsmAI, BsmFI, BsmI, Bso31I, BspCNI, BspMI, BspPI, BspQI, BspTNI, BsrDI, BsrI, Bst6I, BstF5I, BstMAI, BstV1I, BstV2I, BsuI, BtgZI, BtsCI, BtsI-v2, BtsMutI, BveI, CseI, CspCI, Eam11041, EarI, EciI, Eco31I, Eco57I, Esp3I, FaqI, FauI, FokI, GsuI, HgaI, HphI, HpyAV, LguI, LmnI, Lsp1109I, LweI, MboII, MlyI, MmeI, MnlI, Mva1269I, NmeAIII, PaqCI, PciSI, PctI, PleI, PpsI, PsrI, SchI, SfaNI, TaqII, TspDTI and/or TspGWI target sequences.

The method for producing a protected DNA comprising a single-stranded DNA (ssDNA) cassette may comprise:
(a) contacting a precursor dsDNA comprising a first and a second strand with a Type IIS endonuclease, wherein the precursor dsDNA comprises on the first strand a cassette, a Type IIS endonuclease target sequence 5' of the cassette and a Type IIS endonuclease target sequence 3' of the cassette;
(b) digesting the precursor dsDNA with the Type IIS endonuclease to generate a digested precursor dsDNA;
(c) contacting the digested precursor dsDNA with a ligase and first and second adaptor molecules, wherein the first adaptor molecule comprises x nuclease-resistant nucleotides and the second adaptor molecule comprises y nuclease-resistant nucleotides, and wherein x is at least 1 and y is at least 1;
(d) ligating the first adaptor molecule to a first end of the digested precursor dsDNA and ligating the second adaptor molecule to a second end of the digested precursor dsDNA thereby generating a partially protected dsDNA, wherein the partially protected dsDNA comprises a first strand and a second strand, wherein the first strand of the partially protected dsDNA comprises the cassette, x nuclease-resistant nucleotides (from the first adaptor molecule) 5' of the cassette, and y nuclease-resistant nucleotides (from the second adaptor molecule) 3' of the cassette;
(e) contacting the partially protected dsDNA with an exonuclease; and
(f) digesting the second strand of the partially protected dsDNA with the exonuclease thereby generating the protected DNA; optionally wherein steps (a) (i.e. contacting a precursor dsDNA comprising a first and a second strand with a Type IIS endonuclease) to (d) (i.e. ligating the first and second adaptor molecules to the digested precursor dsDNA) are performed in a single contiguous aqueous volume.

The precursor dsDNA may be a product of amplification. Preferably, the amplification is rolling circle amplification.

The method may further comprise, before step (a) (i.e. the step of contacting a precursor dsDNA with the endonuclease), a step of amplifying a DNA template to produce the precursor DNA, wherein the DNA template comprises the cassette and the endonuclease target sequences, optionally wherein the DNA template is amplified by rolling circle amplification.

Thus, the method for producing a protected DNA comprising a single-stranded DNA (ssDNA) cassette may comprise:
(a) amplifying a DNA template to generate a precursor dsDNA, wherein the DNA template comprises a cassette and an endonuclease target sequence 5' of the cassette and an endonuclease target sequence 3' of the cassette, optionally wherein the DNA template molecule is amplified by rolling circle amplification;
(b) contacting the precursor dsDNA comprising a first and a second strand with an endonuclease, wherein the precursor dsDNA comprises on the first strand the cassette, the endonuclease target sequence 5' of the cassette and the endonuclease target sequence 3' of the cassette;
(c) digesting the precursor dsDNA with the endonuclease to generate a digested precursor dsDNA;
(d) contacting the digested precursor dsDNA with a ligase and first and second adaptor molecules, wherein the first and second adaptor molecules each comprise a nuclease-resistant nucleotide;
(e) ligating the first adaptor molecule to a first end of the digested precursor dsDNA and ligating the second adaptor molecule to a second end of the digested precursor dsDNA thereby generating a partially protected dsDNA, wherein the partially protected dsDNA comprises a first strand and a second strand, wherein the first strand of the partially protected dsDNA comprises the cassette, a nuclease-resistant nucleotide (from the first adaptor molecule) 5' of the cassette, and a nuclease-resistant nucleotide (from the second adaptor molecule) 3' of the cassette;
(f) contacting the partially protected dsDNA with an exonuclease; and
(g) digesting the second strand of the partially protected dsDNA with the exonuclease thereby generating the protected DNA.

The method for producing a protected DNA comprising a single-stranded DNA (ssDNA) cassette may comprise:
(a) amplifying a DNA template to generate a precursor dsDNA, wherein the DNA template comprises a cassette and an endonuclease target sequence 5' of the cassette and an endonuclease target sequence 3' of the cassette, optionally wherein the DNA template molecule is amplified by rolling circle amplification;
(b) contacting the precursor dsDNA comprising a first and a second strand with an endonuclease, wherein the precursor dsDNA comprises on the first strand the cassette, the endonuclease target sequence 5' of the cassette and the endonuclease target sequence 3' of the cassette;
(c) digesting the precursor dsDNA with the endonuclease to generate a digested precursor dsDNA;
(d) contacting the digested precursor dsDNA with a ligase and first and second adaptor molecules, wherein the first and second adaptor molecules each comprise at least 3 nuclease-resistant nucleotides;
(e) ligating the first adaptor molecule to a first end of the digested precursor dsDNA and ligating the second adaptor molecule to a second end of the digested precursor dsDNA thereby generating a partially protected dsDNA, wherein the partially protected dsDNA comprises a first strand and a second strand, wherein the first strand of the partially protected dsDNA comprises the cassette, at least 3 nuclease-resistant nucleotides (from the first adaptor molecule) 5' of the cassette, and at least 3 nuclease-resistant nucleotides (from the second adaptor molecule) 3' of the cassette;
(f) contacting the partially protected dsDNA with an exonuclease; and
(g) digesting the second strand of the partially protected dsDNA with the exonuclease thereby generating the protected DNA.

The method for producing a protected DNA comprising a single-stranded DNA (ssDNA) cassette may comprise:
(a) amplifying a DNA template to generate a precursor dsDNA, wherein the DNA template comprises a cassette and an endonuclease target sequence 5' of the cassette and an endonuclease target sequence 3' of the cassette, optionally wherein the DNA template molecule is amplified by rolling circle amplification;
(b) contacting the precursor dsDNA comprising a first and a second strand with an endonuclease, wherein the precursor dsDNA comprises on the first strand the cassette, the endonuclease target sequence 5' of the cassette and the endonuclease target sequence 3' of the cassette;
(c) digesting the precursor dsDNA with the endonuclease to generate a digested precursor dsDNA;
(d) contacting the digested precursor dsDNA with a ligase and first and second adaptor molecules, wherein the first and second adaptor molecules each comprise at least 5 nuclease-resistant nucleotides;
(e) ligating the first adaptor molecule to a first end of the digested precursor dsDNA and ligating the second adaptor molecule to a second end of the digested precursor dsDNA thereby generating a partially protected dsDNA, wherein the partially protected dsDNA comprises a first strand and a second strand, wherein the first strand of the partially protected dsDNA comprises the cassette, at least 5 nuclease-resistant nucleotides (from the first adaptor molecule) 5' of the cassette, and at least 5 nuclease-resistant nucleotides (from the second adaptor molecule) 3' of the cassette;
(f) contacting the partially protected dsDNA with an exonuclease; and
(g) digesting the second strand of the partially protected dsDNA with the exonuclease thereby generating the protected DNA.

The method for producing a protected DNA comprising a single-stranded DNA (ssDNA) cassette may comprise:
(a) amplifying a DNA template to generate a precursor dsDNA, wherein the DNA template comprises a cassette and an endonuclease target sequence 5' of the cassette and an endonuclease target sequence 3' of the cassette, optionally wherein the DNA template molecule is amplified by rolling circle amplification;
(b) contacting the precursor dsDNA comprising a first and a second strand with an endonuclease, wherein the precursor dsDNA comprises on the first strand the cassette, the endonuclease target sequence 5' of the cassette and the endonuclease target sequence 3' of the cassette;
(c) digesting the precursor dsDNA with the endonuclease to generate a digested precursor dsDNA;
(d) contacting the digested precursor dsDNA with a ligase and first and second adaptor molecules, wherein the first adaptor molecule comprises x nuclease-resistant and wherein the second adaptor molecule comprises y nuclease-resistant nucleotides, and wherein x is at least 1 and y is at least 1;
(e) ligating the first adaptor molecule to a first end of the digested precursor dsDNA and ligating the second adaptor molecule to a second end of the digested precursor dsDNA thereby generating a partially protected dsDNA, wherein the partially protected dsDNA comprises a first strand and a second strand, wherein the first strand of the partially protected dsDNA comprises the cassette, x nuclease-resistant nucleotides (from the first adaptor molecule) 5' of the cassette, and y nuclease-resistant nucleotides (from the second adaptor molecule) 3' of the cassette;
(f) contacting the partially protected dsDNA with an exonuclease; and
(g) digesting the second strand of the partially protected dsDNA with the exonuclease thereby generating the protected DNA.

x and y may be independently selected from at least 1, at least 2, at least 3, at least 4 and at least 5. x and y may be different or the same. Preferably x and y are both at least 3. x and y may both be at least 5. x may be 3 and y be 1 and *vice versa.* x may be 3 and y be 5 and *vice versa.*

Steps (b) (i.e. contacting the precursor dsDNA comprising a first and a second strand with an endonuclease) to (e) (i.e. ligating the first and second adaptor molecules to the digested precursor dsDNA) may be performed in a single contiguous aqueous volume.

The step of amplifying a DNA template may be an *in vitro* or *in vivo* amplification. Preferably, the amplification is an *in vitro* amplification. For example, the amplification may be performed by rolling circle amplification (RCA), MALBAC method, traditional polymerase chain reaction (PCR), nucleic acid sequence-based amplification (NASBA), loop-mediated isothermal amplification (LAMP), helicase-dependent amplification (HDA), multiple displacement amplification (MDA) and recombinase polymerase amplification (RPA). Preferably, the amplification is rolling circle amplification.

Rolling circle amplification may be performed without any primers, or in the presence of a primer or multiple primers. For example, the primer may be a synthetic primer. The primers may be random primers. Rolling circle amplification may be performed in the presence of a primase. The primase may be TthPrimPol. Preferably, if the rolling circle amplification is performed without any primers, it is performed in the presence of a primase, such as TthPrimPol. Similarly, if a primer is used during amplification reaction, a primase is not used. The double-stranded DNA product may be generated by the rolling circle amplification in vitro under isothermal conditions using a suitable nucleic acid polymerase, such as Phi29 DNA polymerase.

The method may further comprise a step of purification of the digested precursor dsDNA after digesting the precursor dsDNA.

The method may further comprise a step of purification of the partially protected dsDNA after ligating the first and second adaptor molecules to the digested precursor dsDNA.

The method may further comprise a step of purification of the protected DNA after digesting the second strand of the partially protected dsDNA with an exonuclease.

The method for producing a protected DNA comprising a single-stranded DNA (ssDNA) cassette may comprise:
(a) amplifying a DNA template to generate a precursor dsDNA, wherein the DNA template comprises a cassette and a Type IIS endonuclease target sequence 5' of the cassette and a Type IIS endonuclease target sequence 3' of the cassette, wherein the DNA template molecule is amplified by rolling circle amplification;
(b) contacting the precursor dsDNA comprising a first and a second strand with a Type IIS endonuclease, wherein the precursor dsDNA comprises on the first strand the cassette, the Type IIS endonuclease target sequence 5' of the cassette and the Type IIS endonuclease target sequence 3' of the cassette;
(c) digesting the precursor dsDNA with the Type IIS endonuclease to generate a digested precursor dsDNA;
(d) contacting the digested precursor dsDNA with a ligase and first and second adaptor molecules, wherein the first adaptor molecule comprises x nuclease-resistant and wherein the second adaptor molecule comprises y nuclease-resistant nucleotides, and wherein x is at least 1 and y is at least 1;
(e) ligating the first adaptor molecule to a first end of the digested precursor dsDNA and ligating the second adaptor molecule to a second end of the digested precursor dsDNA thereby generating a partially protected dsDNA, wherein the partially protected dsDNA comprises a first strand and a second strand, wherein the first strand of the partially protected dsDNA comprises the cassette, x nuclease-resistant nucleotides (from the first adaptor molecule) 5' of the cassette, and y nuclease-resistant nucleotides (from the second adaptor molecule) 3' of the cassette;
(f) contacting the partially protected dsDNA with an exonuclease; and
(g) digesting the second strand of the partially protected dsDNA with the exonuclease thereby generating the protected DNA; optionally wherein steps (b) (i.e. contacting the precursor dsDNA comprising a first and a second strand with an endonuclease) to (e) (i.e. ligating the first and second adaptor molecules to the digested precursor dsDNA) are performed in a single contiguous aqueous volume.

Producing protected DNA which is partially double-stranded may involve a partially protected dsDNA with a nuclease-resistant nucleotide in the second strand. Accordingly, it may be necessary to nick the second strand of the partially protected dsDNA to provide an unprotected 3' and/or 5' end nucleotide for the exonuclease. The method for producing a protected DNA comprising a single-stranded DNA (ssDNA) cassette may comprise:
(a) providing a partially protected double-stranded DNA (dsDNA) comprising a first strand and a second strand, wherein the first strand of the partially protected dsDNA comprises a cassette with x nuclease-resistant nucleotides at the 5' end of the cassette or 5' of the cassette, and y nuclease-resistant nucleotides at the 3'-end of the cassette or 3' of the cassette, wherein the second strand of the partially protected dsDNA comprises a sequence complementary to the cassette, y' nuclease-resistant nucleotides at the 5' end of the sequence complementary to the cassette or 5' of the sequence complementary to the cassette, and x' nuclease-resistant nucleotides at the 3'-end of the sequence complementary to the cassette or 3' of the sequence complementary to the cassette, wherein x is at least 1, x' is at least 1, y is at least 1 and y' is at least 1;
(b) nicking the second strand of the partially protected dsDNA between the x' and/or y' nuclease-resistant nucleotides; and
(c) digesting the second strand of the partially protected dsDNA with an exonuclease thereby generating the protected DNA.

Nicking may be performed by contacting the partially protected dsDNA with a nicking endonuclease. The nicking endonuclease may be Nt.BspQI, Nt.CviPII, Nt.BstNBI, Nb.BsrDI, Nb.BtsI, Nt.AlwI, Nb.BbvCI, Nb.BsmI, Nb.BssSI and/or Nt.BsmAI. The second strand of the partially protected dsDNA may comprise a target sequence for the nicking endonuclease. Nicking the second strand of the partially protected dsDNA may be performed prior to digesting the second strand of the partially protected dsDNA with an exonuclease. Alternatively, nicking the second strand of the partially protected dsDNA may be performed at the same time as digesting the second strand of the partially protected dsDNA. Nicking the second strand of the partially protected dsDNA may be performed under the same conditions as digesting the second strand of the partially protected dsDNA or the same conditions as digesting the precursor dsDNA.

The method may be a cell-free method.

Aspects of the invention described above in relation to methods for producing protected DNA apply *mutatis mutandis* to all aspects of the invention described herein.

The endonuclease may be a restriction enzyme endonuclease. The endonuclease may be a Type IIS restriction enzyme. The endonuclease may be any enzyme that recognizes a DNA sequence and cleaves outside of the recognition sequence. For example, the endonuclease may be a BbsI, BsaI, BsmBI, BspQI, BtgZI, Esp3I,SapI, AarI, Acc36IAcIWI, AcuI, AjuI, AloI, AIw26I, AlwI, ArsI, AsuHPI, BaeI, BarI, BbvI, BccI, BceAI, BcgI, BciVI, BcoDI, BfuAI, BfuI, BmrI, BmsI, BmuI, BpiI, BpmI, BpuEI, BsaXI, Bse1I, Bse3DI, BseGI, BseMI, BseMII, BseNI, BseRI, BseXI, BsgI, BsIFI, BsmAI, BsmFI, BsmI, Bso31I, BspCNI, BspMI, BspPI, BspQI, BspTNI, BsrDI, BsrI, Bst6I, BstF5I, BstMAI, BstV1I, BstV2I, BsuI, BtgZI, BtsCI, BtsI-v2, BtsMutI, Bvel, CseI, CspCI, Eam1104I, EarI, EciI, Eco31I, Eco57I, Esp3I, FaqI, FauI, FokI, GsuI, HgaI, HphI, HpyAV, LguI, LmnI, Lsp11091, LweI, MboII, MlyI, MmeI, MnII, Mva1269I, NmeAIII, PaqCI, PciSI, PctI, PleI, PpsI, PsrI, SchI, SfaNI, TaqlI, TspDTI and/or TspGWI restriction enzyme.

Type IIS restriction endonucleases cleave dsDNA outside of the recognition sequence (i.e. an endonuclease target sequence), which means that the recognition sequence (i.e. endonuclease target sequence) is not included in the cleaved product.

The ligase may be a DNA ligase, such as a T4 DNA ligase, T7 DNA ligase, mammalian DNA ligase I, III and IV; Taq DNA ligase, Tth DNA ligase, or *E*. *coli* DNA ligase.

The exonuclease may be a 5' to 3' exonuclease (e.g. exonuclease VIII, RecJf, T7 exonuclease, Lamda exonuclease and/or T5 exonuclease) and/or a 3' to 5' exonuclease (e.g. exonuclease III, exonuclease I and/or exonuclease T). Additionally or alternatively, the exonuclease may have 5' to 3' and 3' to 5' activity (e.g. exonuclease V and/or exonuclease VII).

The exonuclease may digest DNA from one end (i.e. 5' to 3' or 3' to 5'), resulting in protected DNA comprising a protected nucleotide at one end. The exonuclease may digest DNA from both ends (i.e. 5' to 3' and 3' to 5'), resulting in protected DNA comprising a protected nucleotide on both ends.

The exonuclease may be an exonuclease which acts on single-stranded DNA (e.g. *E*. *coli* exonuclease I, exonuclease VII, exonuclease T, RecJf). This may be the case when the partially protected dsDNA is denatured.

Alternatively or in combination, the exonuclease may be an exonuclease which acts on double-stranded DNA (e.g. *E. coli* exonuclease III, T7 exonuclease, exonuclease V, exonuclease VIII, T5 exonuclease, lambda exonuclease). This may be the case when the partially protected dsDNA is not denatured.

The protected DNA may be resistant to 3'-end exonuclease digestion (e.g. by exonuclease III) and/or 5'-end exonuclease digestion (e.g. by exonuclease VIII). The protected DNA may be resistant to 3'-end exonuclease digestion (e.g. by exonuclease III, exonuclease I, exonuclease T, exonuclease V and/or exonuclease VII) and/or 5'-end exonuclease digestion (e.g. by exonuclease VIII, RecJf, T7 exonuclease, Lamda exonuclease, T5 exonuclease, exonuclease V and/or exonuclease VII).

As used herein the term "protected nucleotide" or "nuclease-resistant nucleotide" is intended to encompass any type of molecule that provides or enhances resistance to nuclease digestion (especially exonuclease digestion). Although the adaptor molecules are described herein as comprising phosphorothioated nucleotides, the skilled person would appreciate that the adaptor molecules may instead comprise any molecules that provide resistance to exonuclease digestion. For example, the adaptor molecules may comprise nuclease resistant nucleotides i.e. modified nucleotides that provide or increase resistance to nucleases (e.g. exonucleases). The adaptor molecules may comprise a peptide, polypeptide or protein that provides or increases resistance to nucleases (e.g. exonucleases) digestion. The adaptor molecules may comprise 2'-O-methyl nucleotides or 2'-O-methoxyethyl (MOE) nucleotides.

As used herein, the term "phosphorothioated nucleotide" refers to a nucleotide that has an altered phosphate backbone, wherein, the sugar moieties are linked by a phosphorothioate bond. In the phosphate backbone of an oligonucleotide sequence, the phosphorothioate bond contains a sulphur atom as a substitute for a non-bridging oxygen atom. This modification renders the internucleotide linkage resistant to nuclease degradation.

The step of digesting the precursor dsDNA with the endonuclease to generate a digested precursor dsDNA may be performed by incubating the combined components under conditions that promote digestion of the precursor dsDNA to produce the digested precursor dsDNA. The digestion of the precursor dsDNA to produce the digested precursor dsDNA may be performed at a first temperature of 1°C-100°C, 1°C -80°C, 5°C-70°C, 10°C-60°C, 15°C-55°C, 20°C-50°C, 25°C -45°C, 30°C-40°C, 35°C-39°C, 36°C-38°C, or at about 37°C. The digestion may be endonuclease digestion, preferably Type IIS endonuclease digestion.

The step of ligating the first and second adaptor molecules to the digested precursor dsDNA may be performed by incubating the combined components under conditions that promote ligation of the first and second adaptor molecules to the digested precursor dsDNA to produce the partially protected dsDNA. The ligating may be performed by creating a covalent link between the first and/or second adaptor molecule and the first and/or second end of the digested precursor dsDNA.

The ligation may be at least 5%, at least 10%, at least 15, at least 20%, at least 25%, at least 30%, at least 35%, at least 40, at least 45%, at least 50%, at least 55%, at least 60, at least 65, at least 70%, at least 75, at least 80%, at least 82%, at least 85%, at least 90%, or at least 95% efficient. For example, at least 5%, at least 10%, at least 15, at least 20%, at least 25%, at least 30%, at least 35%, at least 40, at least 45%, at least 50%, at least 55%, at least 60, at least 65, at least 70%, at least 75, at least 80%, at least 82%, at least 85%, at least 90%, or at least 95% of the digested precursor dsDNA may be incorporated into partially protected dsDNA. Preferably, the ligation is at least 15% efficient.

Ligation efficiency may be established based on DNA quantification values before and after the digestion/ligation reaction. Thus, ligation efficiency may be established based on the equation: (starting amplified DNA amount) / (final linear DNA amount) x 100%.

Ligation efficiency may also be established based on DNA quantification values before and after the digestion/ligation reaction and the subsequent exonuclease treatment to remove remaining open DNA constructs and adaptor molecules excess.

For example, the precursor dsDNA generated by the amplification (e.g. rolling circle amplification) is first quantified so that the amount of the precursor dsDNA used as the starting material during the digestion/ligation reaction is known. After all the enzymatic reactions, the partially protected dsDNA is quantified to calculate the ligation efficiency as per the equation above.

DNA quantification methods are known to a person skilled in the art. For example, DNA quantifications may be carried out using the Qubit dsDNA BR assay from ThermoFisher (https://www.thermofisher.com/order/catalog/product/Q32850#/Q32850).

The step of ligating the digested precursor dsDNA to the first and second adaptor molecules may be performed at a second temperature of 1°C -90°C, 2°C -70°C, 5°C-60°C, 8°C-55°C, 9°C-50°C, 10°C-45°C, 11°C-40°C, 12°C-37°C, 13°C-30°C, 14°C-25°C, 15°C-20°C or at about 16°C.

Using the above conditions the endonuclease may be a Type IIS restriction endonuclease (e.g. Bsal) and the ligase may be T4 DNA ligase, T7 DNA ligase, mammalian DNA ligase I, III and IV; Taq DNA ligase, Tth DNA ligase, or *E*. *coli* DNA ligase.

The step of digesting the second strand of the partially protected dsDNA with an exonuclease may be performed at a temperature of 5-90°C, 10-80°C, 15-70°C, 20-60°C, 25-50°C, 30-45°C or 35-40°C. Preferably, the step of digesting the second strand of the partially protected dsDNA with an exonuclease is performed at a temperature of 30-45°C. The step of digesting the second strand of the partially protected dsDNA with an exonuclease may be performed for at least 10, at least 20, at least 30, at least 40, at least 50, at least 60 or at least 120 minutes. Preferably, the step of digesting the second strand of the partially protected dsDNA with an exonuclease may be performed for at least 60 minutes. For example, the step of digesting the second strand of the partially protected dsDNA with an exonuclease may be performed at around 37 °C for at least 120 minutes.

The step of digesting the second strand of the partially protected dsDNA with an exonuclease may be followed by inactivating the exonuclease, for example by heat-inactivation. Thus, the step of inactivating the exonuclease may be performed at a temperature of 60-90°C, 65-85°C or 70-80°C. Preferably, the step of inactivating the exonuclease is performed at a temperature of 70-80°C. The step of inactivating the exonuclease may be performed at a temperature of at least 60°C, at least 65°C, at least 70°C or at least 80°C. Preferably, the step of inactivating the exonuclease is performed at a temperature of at least 70°C. The step of inactivating the exonuclease may be performed for at least 1, at least 5, at least 10, at least 20 or at least 30 minutes. Preferably, the step of inactivating the exonuclease is performed for at least 5 minutes.

The step of digesting the second strand of the partially protected dsDNA with an exonuclease may be performed at a temperature of 30-45°C for at least 60 minutes followed by performing the step of inactivating the exonuclease at a temperature of at least 70°C for at least 5 minutes.

The cassette may comprise at least 25, at least 50, at least 100, at least 150, at least 200, at least 210, at least 220, at least 230, at least 240, at least 250, at least 260, at least 270, at least 280, at least 290, at least 300, at least 325, at least 350, at least 375, at least 400, at least 450, at least 500, at least 600, at least 700, at least 800, at least 900, at least 1000, at least 2000, at least 3000, at least 4000, at least 5000, at least 6000, at least 7000, at least 8000, at least 9000, at least 10,000, at least 11,000, at least 12,000, at least 13,000, at least 14,000, or at least 15,000 nucleotides. Preferably, the cassette comprises at least 100 nucleotides.

The cassette may be a single cassette. The term "single cassette" as used herein is intended to encompass a product that does not comprise or consist of a plurality of cassettes. That is to say that the protected DNA (or partially protected dsDNA or precursor dsDNA) comprises only a single cassette, which may comprise a single coding sequence of a gene of interest. The single cassette may not comprise or consist of a plurality of tandem repeat sequences, and/or concatemeric DNA. The term "single cassette" as used herein is intended to encompass a single copy of the DNA sequence of interest, for example, a single copy of the coding sequence. Thus, the "single cassette" may not encompass a cassette that comprises or consist of multiple copies of the same DNA sequence linked in series. The single cassette may comprise a collection of genes of interest. For example, the single cassette may comprise the sequence for at least two, three, four, or five genes of interest. The genes of interest may not be the same in a single cassette.

The cassette may comprise at least 25, at least 50, at least 100, at least 150, at least 200, at least 210, at least 220, at least 230, at least 240, at least 250, at least 260, at least 270, at least 280, at least 290, at least 300, at least 325, at least 350, at least 375, at least 400, at least 450, at least 500, at least 600, at least 700, at least 800, at least 900, at least 1000, at least 2000, at least 3000, at least 4000, at least 5000, at least 6000, at least 7000, at least 8000, at least 9000, at least 10,000, at least 11,000, at least 12,000, at least 13,000, at least 14,000, or at least 15,000 nucleotides. Preferably, the cassette comprises at least 100 nucleotides.

The cassette may comprise a coding sequence. The coding sequence may encode a gene of interest, for example a gene encoding a protein. The gene may comprise at least 200, at least 500, at least 1000, least 1500, at least 2000, at least 3000, at least 4000, at least 5000, at least 7500, at least 10,000 nucleotides. Preferably, the gene comprises at least 200 nucleotides.

The cassette may comprise at least a portion of a promoter and a coding sequence. The cassette may comprise a promoter and a coding sequence. The cassette may comprise a promoter, a coding sequence, a ribosomal binding site and a translational termination sequence. The cassette may additionally comprise sequences aiding protein expression, such as a cap-independent translation element. The cassette may comprise (or encode) a repair template (or editing template). The repair template (or editing template) may be for use in CRISPR-Cas mediated homology directed repair (HDR). The cassette may encode CRISPR guide RNA. The cassette may be a mammalian expression cassette. The promoter may be a CMV promoter. The cassette may further comprise an enhancer. The cassette may further comprise a reporter gene, such as an eGFP reporter gene or a luciferase reporter gene. The cassette may further comprise a homopolymeric sequence, such as a polyA, poly C, polyT or polyG sequence. The homopolymeric sequence may be between 3-200 nucleotides in length. The homopolymeric sequence may be used to facilitate purification of the cassette, in which case, the homopolymeric sequence may be between 4-12 nucleotides in length, or between 5-10 nucleotides in length. The homopolymeric sequence may be used to improve mRNA expression, in which case, the homopolymeric sequence may be between 10-200 nucleotides in length, preferably between 80-150 nucleotides in length. The homopolymeric sequence may be at least 10, at least 20, at least 30, at least 40, at least 50, at least 60, at least 70, at least 80, at least 90, at least 100, at least 110, at least 120, at least 130, at least 140, at least 150, at least 160, at least 170, at least 180, at least 190, or at least 200 nucleotides in length. For example, the homopolymeric sequence may comprise a polyA sequence of at least 120 nucleotides.

The first and second adaptor molecules may be nucleic acids, optionally DNA. The first and second adaptor molecules may comprise a first strand hybridised to a second strand (e.g. dsDNA).

The first and second adaptor molecules may have different sequences.

The first and second adaptor molecules may comprise dsDNA comprising a first strand and a second strand. The first strands of the first and second adaptor molecules may be ligated by the ligase to the first strand of the digested precursor dsDNA molecule and the second strands of the first and second adaptor molecules may be ligated by the ligase to the second strand of the digested precursor dsDNA molecule.

The first adaptor molecule may be compatible with the first end of the digested precursor dsDNA and incompatible with the second end of the digested precursor dsDNA. The second adaptor molecule may be compatible with the second end of the digested precursor dsDNA and incompatible with the first end of the digested precursor dsDNA.

The first adaptor molecule may be compatible with the 5' end of the first strand of the digested precursor dsDNA and incompatible with the 3' end of the first strand of the digested precursor dsDNA. The second adaptor molecule may be compatible with the 3' end of the first strand of the digested precursor dsDNA and incompatible with the 5' end of the first strand of the digested precursor dsDNA.

The first strand of the first adaptor molecule may be compatible with the 5' end of the first strand of the digested precursor dsDNA and incompatible with the 3' end of the first strand and the 3' and 5' ends of the second strand of the digested precursor dsDNA. The second strand of the first adaptor molecule may be compatible with the 3' end of the second strand of the digested precursor dsDNA and incompatible with the 5' end of the second strand and the 3' and 5' ends of the first strand of the digested precursor dsDNA.

The first strand of the second adaptor molecule may be compatible with the 3' end of the first strand of the digested precursor dsDNA and incompatible with the 5' end of the first strand and the 3' and 5' ends of the second strand of the digested precursor dsDNA. The second strand of the second adaptor molecule may be compatible with the 5' end of the second strand of the digested precursor dsDNA and incompatible with the 3' end of the second strand and the 3' and 5' ends of the first strand of the digested precursor dsDNA.

The second strand of the first and second adaptor molecules may not be resistant to exonuclease digestion. The second strand of the first and second adaptor molecules may be susceptible to exonuclease digestion. The second strand of the first and second adaptor molecules may not comprise any nuclease-resistant nucleotides.

The first strand of the first adaptor molecule may comprise the x nucleotide-resistant nucleotides and the first strand of the second adaptor molecule may comprise the y nucleotide-resistant nucleotides. The nucleotide-resistant nucleotide(s) in the first strand of the first adaptor molecule may be located at the 5' or 3' end, preferably the 5' end; and/or the nucleotide-resistant nucleotide(s) in the first strand of the second adaptor molecule may be located at the 5' or 3' end, preferably the 3' end. The nuclease-resistant nucleotide may be a phosphorothioated nucleotide.

The second strand of the first adaptor molecule may comprise the x' nucleotide-resistant nucleotides and the second strand of the second adaptor molecule may comprise the y' nucleotide-resistant nucleotides. The nucleotide-resistant nucleotide(s) in the second strand of the first adaptor molecule may be located at the 5' or 3' end, preferably the 3' end; and/or the nucleotide-resistant nucleotide(s) in the second strand of the second adaptor molecule may be located at the 5' or 3' end, preferably the 5' end. The nuclease-resistant nucleotide may be a phosphorothioated nucleotide.

The first adaptor molecule and/or the second adaptor molecule may be a synthetic adaptor molecule.

The first adaptor molecule and/or the second adaptor molecule may comprise a self-complementary element which creates a loop, such as a hairpin loop or a stem loop. Thus, the first adaptor molecule may comprise a hairpin or a stem-loop. The second adaptor molecule may comprise a hairpin or a stem-loop. Both the first and second adaptor molecules may comprise a hairpin or a stem-loop. The adaptor molecules may each comprise a double-stranded portion comprising a first strand and a second strand, wherein the first strand and the second strand are linked together in a hairpin such that the first strand is hybridized to the second strand. The double-stranded portion of an adaptor may comprise a 3' overhang or a 5' overhang of at least 1, at least 2, at least 3, at least 4, or at least 5 nucleotides. Preferably the 3' overhang or the 5' overhang is 4-8 nucleotides. Each end of the digested precursor dsDNA may comprise a 3' or a 5' overhang. A portion of the first adaptor molecule (e.g. the overhang) may be complementary to the first end of the digested precursor dsDNA. A portion of the second adaptor molecule may be complementary to the second end of the digested precursor dsDNA.

If the first and/or second adaptor molecules comprise a hairpin, following ligation of the first and second adaptor molecules to the digested precursor dsDNA there may be a hairpin 3' and/or 5' of the cassette. The hairpin may link the first and second strands of the digested precursor dsDNA to form a closed loop at one or both ends. Thus, the methods described herein may further comprise nicking a closed loop at one or both ends in order to generate a partially protected dsDNA with a 3' and/or 5' end nucleotide on the second strand.

The first adaptor molecule and/or the second adaptor molecule may comprise an overhang. The first and/or second ends of the digested precursor dsDNA may comprise a 3' or a 5' overhang. A portion of the first adaptor molecule (e.g. the overhang) may be complementary to the first end of the digested precursor dsDNA. A portion of the second adaptor molecule (e.g. the overhang) may be complementary to the second end of the digested precursor dsDNA.

The first adaptor molecule may comprise an overhang which is complementary to the first end of the digested precursor dsDNA. The second adaptor molecule may comprise an overhang which is complementary to the second end of the digested precursor dsDNA.

The first adaptor molecule may comprise an overhang which is non-complementary to the second end of the digested precursor dsDNA. The second adaptor molecule may comprise an overhang which is non-complementary to the first end of the digested precursor dsDNA.

The first adaptor molecule may comprise an overhang complementary to an overhang at the first end of the digested precursor dsDNA and the second adaptor molecule may comprise an overhang complementary to an overhang at the second end of the digested precursor dsDNA. Optionally, the overhang of the first adaptor molecule may not be complementary to the overhang at the second end of the digested precursor dsDNA and the overhang of the second adaptor molecule may not be complementary to the overhang at the first end of the digested precursor dsDNA.

The first adaptor molecule may comprise an overhang that is complementary to and anneals to a 5' overhang of the digested precursor dsDNA and the second adaptor molecule may comprise an overhang that is complementary to and anneals to a 3' overhang of the digested precursor dsDNA.

The first adaptor molecule may comprise an overhang that is complementary to and anneals to a 3' overhang of the digested precursor dsDNA and the second adaptor molecule may comprise an overhang that is complementary to and anneals to a 5' overhang of the digested precursor dsDNA.

The first adaptor molecule and/or the second adaptor molecule may not be a plasmid or a vector DNA.

The first adaptor molecule and/or the second adaptor molecule may comprise a single-stranded portion. The single-stranded portion may form a hairpin or a stem-loop. Thus, the first adaptor molecule and/or the second adaptor molecule may comprise a loop portion. The single-stranded portion may comprise less than 10, 9, 8, 7, 6, 5, 4, 3, 2 nucleotides. Preferably, the single-stranded portion comprises 5 nucleotides.

The first adaptor molecule and/or the second adaptor molecule may comprise a double-stranded portion. The double-stranded portion may comprise less than 50, less than 45, less than 40, less than 35, less than 30, less than 25, less than 20, less than 15, or less than 10 base pairs. The double-stranded portion may comprise at least 5, at least 6, at least 7, at least 8, at least 9, at least 10, at least 11, at least 12, at least 13, at least 14, or at least 15 base pairs.

The first adaptor molecule and/or the second adaptor molecule may comprise a 5' phosphate. The 5' phosphate may facilitate ligation to the digested precursor dsDNA (which may comprise a 3'-OH group at first and/or second ends). The first adaptor molecule and/or the second adaptor molecule may comprise a 3'-OH. The 3'-OH may facilitate ligation to the digested precursor dsDNA (which may comprise a 5' phosphate at first and/or second ends).

The first strand of the first adaptor molecule may comprise the sequence of SEQ ID NO: 7 or a portion thereof. The second strand of the first adaptor molecule may comprise the sequence of SEQ ID NO: 8 or 9 or a portion thereof. The first strand of the second adaptor molecule may comprise the sequence of SEQ ID NO: 10 or a portion thereof. The second strand of the second adaptor molecule may comprise the sequence of SEQ ID NO: 11 or 12 or a portion thereof. The first and second adapter molecules may comprise a different nucleic acid sequence.

The first adaptor molecule may comprise a portion that is complementary to the first end of the digested precursor dsDNA. The second adaptor molecule may comprise a portion that is complementary to the second end of the digested precursor dsDNA. The first adaptor molecule may comprise a portion that anneals to the first end of the digested precursor dsDNA. The second adaptor molecule may comprise a portion that anneals to the second end of digested precursor dsDNA. The first adaptor molecule may comprise a portion that is complementary and anneals to the first end of the digested precursor dsDNA. The second adaptor molecule may comprise a portion that is complementary and anneals to the second end of the digested precursor dsDNA.

The portion that is complementary or anneals to the first or second end of the digested precursor dsDNA may be a 5' overhang or a 3' overhang of the first and/or second adaptor molecule. The overhang of the first adaptor molecule may be complementary to the first end of the digested precursor dsDNA and/or the overhang of the second adaptor molecule may be complementary to the second end of the digested precursor dsDNA. The overhang of the first adaptor molecule may anneal to the first end of the digested precursor dsDNA and/or the overhang of the second adaptor molecule may anneal to the second end of the digested precursor dsDNA. The overhang of the first adaptor molecule may be complementary to and anneal to the first end of the digested precursor dsDNA and/or the overhang of the second adaptor molecule may be complementary to and anneal to the second end of the digested precursor dsDNA.

The first adaptor molecule may be appended to a first end of the digested precursor dsDNA and the second adaptor molecule may be appended to a second end of the digested precursor dsDNA. The appending of the first adaptor molecule and/or the second adaptor molecule may be performed by hybridization or ligation of the adaptor molecules to the ends of the digested precursor dsDNA. Thus, the first adaptor molecule may be hybridized to the first end of the digested precursor dsDNA. The second adaptor molecule may be hybridized to the second end of the digested precursor dsDNA. The first adaptor molecule may be ligated to the first end of the digested precursor dsDNA. The second adaptor molecule may be ligated to the second end of the digested precursor dsDNA. The appending of the first adaptor molecule and the second adaptor molecule may be performed by both hybridization and ligation of the adaptor molecules to the ends of the digested precursor dsDNA. Thus, the first adaptor molecule may be hybridized and ligated to the first end of the digested precursor dsDNA. The second adaptor molecule may be hybridized and ligated to the second end of the digested precursor dsDNA. The hybridization is based on complementarity of a portion of the first and/or second adaptor molecules to the first and/or second end of the digested precursor dsDNA.

The first adaptor molecule and/or the second adaptor molecule may not comprise a Type IIS endonuclease target sequence. The first adaptor molecule and/or the second adaptor molecule may not comprise BbsI, BsaI, BsmBI, BspQI, BtgZI, Esp3I,SapI, AarI, Acc36IAcIWI, AcuI, AjuI, AloI, AIw26I, AlwI, ArsI, AsuHPI, BaeI, BarI, BbvI, BccI, BceAI, BcgI, BciVI, BcoDI, BfuAI, BfuI, BmrI, BmsI, BmuI, BpiI, BpmI, BpuEI, BsaXI, Bse1I, Bse3DI, BseGI, BseMI, BseMII, BseNI, BseRI, BseXI, BsgI, BsIFI, BsmAI, BsmFI, BsmI, Bso31I, BspCNI, BspMI, BspPI, BspQI, BspTNI, BsrDI, BsrI, Bst6I, BstF5I, BstMAI, BstV1I, BstV2I, BsuI, BtgZI, BtsCI, BtsI-v2, BtsMutI, BveI, CseI, CspCI, Eam1104I, EarI, EciI, Eco31I, Eco57I, Esp3I, FaqI, FauI, FokI, GsuI, HgaI, HphI, HpyAV, LguI, LmnI, Lsp11091, LweI, MbolI, MlyI, MmeI, MnlI, Mva1269I, NmeAIII, PaqCI, PciSI, PctI, PleI, PpsI, PsrI, SchI, SfaNI, TaqlI, TspDTI and/or TspGWI target sequences.

The first adaptor molecule and/or the second adaptor molecule may comprise one or more locked nucleic acids (LNAs).

The first adaptor molecule and/or the second adaptor molecule may confer resistance to the nuclease digestion, such as exonuclease digestion (e.g. exonuclease III or VIII digestion).

The first adaptor molecule and/or the second adaptor molecule may comprise one or more protected nucleotides (i.e. nuclease-resistant nucleotides), such as phosphorothioated nucleotides. The protected nucleotides may be located in the single-stranded portion (e.g. hairpin portion) or the double-stranded portion. The protected nucleotides may be located in the overhang portion of the adaptor molecules.

The first and second adaptor molecules may comprise one or more phosphorothioated nucleotides, such that, once the adaptor molecules are appended (e.g. ligated) to the digested precursor dsDNA and the second strand of the partially protected dsDNA is digested by the exonuclease, the protected DNA is resistant to nuclease digestion or has improved or enhanced resistance to nuclease digestion. The protected DNA product may be resistant to 3'-end exonuclease digestion (e.g. by exonuclease III) and/or 5'-end exonuclease digestion (e.g. by exonuclease VIII).

The adaptor molecule may comprise a plurality of phosphorothioated nucleotides. For example, the adaptor molecules may comprise at least 2, at least 3, at least 4, at least 5, at least 6, at least 7, at least 8, at least 9, at least 10, at least 11, at least 12, at least 13, at least 14, at least 15 or at least 16 phosphorothioated nucleotides in each strand.

The adaptor molecule may be a nucleic acid adaptor molecule. The adaptor molecule may be double-stranded. The adaptor molecule may comprise a portion that is double-stranded.

The first and/or second adaptor molecules may comprise at least 3, at least 4, at least 5, at least 6, at least 7, at least 8, at least 9, at least 10, at least 11, at least 12, at least 13, at least 14, at least 15, or at least 16 base pairs.

The adaptor molecule may comprise a plurality of phosphorothioated nucleotides at internal positions in each strand. For example, the adaptor molecules may comprise at least 1, at least 2, at least 3, at least 4, at least 5, at least 6, at least 7, at least 8, at least 9, at least 10, at least 11, at least 12, at least 13, at least 14, at least 15 or at least 16 phosphorothioated nucleotides at internal positions in each strand. Preferably, the adaptor molecule comprises at least 2 phosphorothioated nucleotides at internal positions in each strand.

The internal positions may not be located between the second and penultimate nucleotide of the adaptor molecule. The internal positions may be any position in the adaptor molecules other than the last nucleotide at the end of each strand.

The adaptor molecule may comprise at least 5%, at least 10%, at least 20%, at least 30%, at least 40%, at least 50%, at least 60%, at least 70%, at least 80%, at least 90%, at least 95%, or at least 100% of protected nucleotides.

Once the adaptor molecules are appended to the digested precursor dsDNA and the partially protected dsDNA is digested by the exonuclease, the protected DNA may comprise a protected nucleotide at the 5' end of the ssDNA cassette or 5' of the ssDNA cassette, and a nuclease-resistant nucleotide at the 3'-end of the ssDNA cassette or 3' of the ssDNA cassette.

The nucleotides resistant to exonuclease digestion (i.e. protected nucleotides) suitable for use in the present invention may be phosphorothioated nucleotides. For example, phosphorothioated nucleotides may be α-S-dATP (i.e. 2'-deoxyadenosine-5'-(α-thio)-triphosphate), α-S-dCTP (i.e. 2'-deoxycytidine-5'-(a-thio)-triphosphate), α-S-dGTP (i.e. 2'-deoxyguanosine-5'-(α-thio)-triphosphate), α-S-dTTP (i.e. 2'-deoxythymidine-5'-(α-thio)-triphosphate), α-S-dUTP (i.e. 2'-deoxyuridine-5'-(α-thio)-triphosphate), and/or uridine 2', 3'-cyclophosphorothioate.

The phosphorothioated nucleotides may be Sp-isomers, Rp-isomers or a mixture of both Sp- and Rp-isomers.

The nucleotides resistant to exonuclease digestion (i.e. protected nucleotides) may be MOE nucleotides of at least one type, or at least two, three or four types. For example, the MOE nucleotides may be 2'-O-methoxy-ethyl guanosine, 2'-O-methoxy-ethyl cytidine, 2'-O-methoxy-ethyl adenosine, and/or 2'-O-methoxy-ethyl thymidine.

The adaptor molecules may comprise at least two types of phosphorothioated nucleotides. For example, the at least two types of phosphorothioated nucleotides are: α-S-dATP and α-S-dCTP, α-S-dATP and α-S-dGTP, α-S-dATP and α-S-dTTP, α-S-dCTP and α-S-dGTP, α-S-dCTP and α-S-dTTP, or α-S-dGTP and α-S-dTTP.

The adaptor molecules may comprise at least three types of phosphorothioated nucleotides. For example, the at least three types of phosphorothioated nucleotides are:
(a) α-S-dATP, α-S-dCTP and α-S-dGTP;
(b) α-S-dATP, α-S-dCTP and α-S-dTTP;
(c) α-S-dATP, α-S-dGTP and α-S-dTTP; or
(d) α-S-dCTP, α-S-dGTP and α-S-dTTP.

The adaptor molecules may comprise at least four types of phosphorothioated nucleotides. For example, the at least four types of protected nucleotides are α-S-dATP, α-S-dCTP, α-S-dGTP and α-S-dTTP.

The first adaptor molecule and/or the second adaptor molecule may comprise a functional portion. The functional portion may be a binding molecule, a targeting sequence, or a probe.

The functional portion may be a probe. As used herein, the term "probe" refers to a fragment of DNA, RNA or DNA/RNA chimera of variable length (e.g. 3-1000 bases long), which is used to detect the presence of target nucleotide sequences that are complementary to the sequence in the probe. Typically, the probe hybridizes to single-stranded nucleic acid whose base sequence allows probe-target base pairing due to complementarity between the probe and target. Thus, the functional portion may be a DNA sequence, a RNA sequence or a DNA/RNA chimera sequence. As used herein, the term "complementary" refers to the pairing of nucleotide sequences according to Watson/Crick pairing rules. For example, a sequence 5'-GCGGTCCCA-3' has the complementary sequence of 5'-TGGGACCGC-3'. A complement sequence can also be a sequence of RNA complementary to the DNA sequence.

The functional portion may be a binding molecule. The term "binding molecule" refers to any molecule capable of binding to the protected DNA described herein and/or that is capable of binding to a further molecule or target. The binding molecule may be a protein, a polypeptide, or a peptide. The binding molecule may be an antibody, such as a monoclonal antibody or a polyclonal antibody. The binding molecule may be an antibody fragment.

The functional portion may facilitate detection of the DNA product by binding to capture molecules (e.g. capture antibodies bound by protein-protein interactions). The functional portion may bind to a cell target, for example, a cell receptor.

The functional portion may be a label. The 'label' can be any chemical entity which enable the detection of the double-stranded nucleic acid molecule via, physical, chemical and/or biological means. The label may be a chromophore, a fluorophore and/or a radioactive molecule.

The functional portion may be a targeting sequence. The targeting sequence may be a fragment of DNA or RNA of variable length, which is used to target the DNA product to a specific location in a cell. The targeting sequence may be used to increased transfection efficiency of non-viral gene delivery by virtue of enhanced nuclear import of the protected DNA. For example, the targeting sequence may be a DNA nuclear targeting sequences (i.e. a recognition sequence for endogenous DNA-binding proteins), such as SV40 enhancer sequence (preferably downstream from the cassette).

To facilitate detection and/or quantification of the protected DNA product, the functional portion may comprise a fluorophore, a radioactive compound or a barcode.

A signal corresponding to the presence, absence and/or level of the protected DNA may be measured using a barcode. The barcode may comprise at least one binding moiety linked to a barcoded portion, wherein the barcoded portion comprises at least one nucleotide (i.e. wherein the barcoded portion comprises a nucleotide sequence at least one nucleotide in length), and wherein the binding moiety is capable of binding to the protected DNA. The binding moiety is capable of binding to 3' and/or 5' end of the protected DNA. The signal may be measured by determining the presence, absence and/or level of the barcoded portion of the barcode (e.g. by sequencing or PCR). The barcoded portion may comprise at least 2, at least 3, at least 4, at least 5, at least 6, at least 7, at least 8, at least 9, or at least 10 nucleotides. The barcode may comprise at least 2 binding moieties (e.g. a first binding moiety and a second binding moiety). For example, the first binding moiety linked to the first barcoded portion may bind to the 3' end of the protected DNA and the second binding moiety linked to the second barcoded portion may bind to the 5' end of the protected DNA. The 3' and 5' ends may comprise a 3' overhang, a 5' overhang or a blunt end.

A signal corresponding to the presence, absence and/or level of the protected DNA may be measured using a fluorophore (i.e. a fluorescently-labelled molecule), which is attached or bound to the protected DNA. The signal may be measured by flow cytometry and/or fluorescence-activated cell sorting.

The functional portion may also facilitate DNA sequencing. For example, the functional portion may be a sequencing adapter. The term "sequencing adapter" is intended to encompass one or more nucleic acid domains that include at least a portion of a nucleic acid sequence (or complement thereof) utilized by a sequencing platform of interest, such as a sequencing platform provided by Illumina^{®} (e.g. the HiSeq^{™}, MiSeq^{™} and/or Genome Analyzer^{™} sequencing systems), Oxford Nanopore^{™} Technologies (e.g. the MinION sequencing system), Ion Torrent^{™} (e.g. the Ion PGM^{™} and/or Ion Proton^{™} sequencing systems), Pacific Biosciences (e.g. the PACBIO RS II sequencing system); Life Technologies^{™} (e.g. a SOLiD sequencing system), Roche (e.g. the 454 GS FLX+ and/or GS Junior sequencing systems), or any other sequencing platform of interest.

The first adaptor molecule and/or the second adaptor molecule may comprise an aptamer.

The first adaptor molecule and/or the second adaptor molecule may be a synthetic adaptor molecule.

The first adaptor molecule and/or the second adaptor molecule may not be a plasmid or a vector DNA.

The first adaptor molecule and/or the second adaptor molecule may comprise a single-stranded portion. The single-stranded portion may comprise less than 10, 9, 8, 7, 6, 5, 4, 3, 2 nucleotides. Preferably, the single-stranded portion comprises 5 nucleotides.

The first adaptor molecule and/or the second adaptor molecule may comprise a double-stranded portion. The double-stranded portion may comprise less than 50, less than 45, less than 40, less than 35, less than 30, less than 25, less than 20, less than 15, or less than 10 base pairs. The double-stranded portion may comprise at least 5, at least 6, at least 7, at least 8, at least 9, at least 10, at least 11, at least 12, at least 13, at least 14, or at least 15 base pairs.

The first adaptor molecule and/or the second adaptor molecule may comprise a 5' phosphate. The 5' phosphate may facilitate ligation to the digested precursor dsDNA.

The first adaptor molecule may comprise a portion that is complementary to the first end of digested precursor dsDNA. The second adaptor molecule may comprise a portion that is complementary to the second end of the digested precursor dsDNA. The first adaptor molecule may comprise a portion that anneals to the first end of the digested precursor dsDNA. The second adaptor molecule may comprise a portion that anneals to the second end of the digested precursor dsDNA.

The first adaptor molecule and/or the second adaptor molecule may comprise an overhang. For example, the first adaptor molecule and/or the second adaptor molecule may comprise a 5' overhang or a 3' overhang. The first adaptor molecule and/or the second adaptor molecule may comprise a blunt end. The overhang may have at least 3 nucleotides (preferably from 4 to 6 nucleotides). The overhang of the first adaptor molecule and/or the second adaptor molecule may be complementary to the first and/or second end of the digested precursor dsDNA. The overhang of the first adaptor molecule and/or the second adaptor molecule may anneal to the first and/or second end of the digested precursor dsDNA.

The partially protected dsDNA may be generated from the precursor dsDNA by performing the steps described herein in a single reaction (i.e. as a single step) in a single contiguous aqueous volume. For example, the steps of contacting a precursor dsDNA with an endonuclease, digesting the precursor dsDNA with the endonuclease to generate a digested precursor dsDNA, contacting the digested precursor dsDNA with a ligase and first and second adaptor molecules, and ligating the first and second adaptor molecules to the digested precursor DNA thereby generating the partially protected dsDNA, may be performed in a single reaction (i.e. as a single step) in a single contiguous aqueous volume. Thus, the partially protected dsDNA may be generated by incubating the precursor dsDNA with the endonuclease, the ligase and first and second adaptor molecules in a single reaction (i.e. a single step) in a single contiguous aqueous volume. The single contiguous aqueous volume may be incubated in order to allow the required digestion and ligation.

The single contiguous aqueous volume may be incubated to generate the partially protected dsDNA by digesting the precursor dsDNA and ligating the first and second adaptor molecules to the digested precursor dsDNA.

The step of incubating the single contiguous aqueous volume may be performed under conditions that promote ligation of the first and second adaptor molecules to the digested precursor dsDNA to produce the partially protected dsDNA. The appending may be performed by creating a covalent link between the first and/or second adaptor molecule and the end(s) of the digested precursor dsDNA.

The step of incubating the single contiguous aqueous volume may be performed under conditions that promote digestion of the precursor dsDNA to produce the digested precursor dsDNA. The digestion of the precursor dsDNA to produce the digested precursor dsDNA may be performed at a first temperature of 1°C-100°C, 1°C -80°C, 5°C-70°C, 10°C-60°C, 15°C-55°C, 20°C-50°C, 25°C -45°C, 30°C-40°C, 35°C-39°C, 36°C-38°C, or at about 37°C. The digestion may be endonuclease digestion, preferably Type IIS endonuclease digestion.

The step of incubating the single contiguous aqueous volume may be performed under conditions that promote ligation of the digested precursor dsDNA to the first and second adaptor molecules. The ligation may be at least 5%, at least 10%, at least 15, at least 20%, at least 25%, at least 30%, at least 35%, at least 40, at least 45%, at least 50%, at least 55%, at least 60, at least 65, at least 70%, at least 75%, at least 80%, at least 82%, at least 85%, at least 90%, or at least 95% efficient. For example, at least 5%, at least 10%, at least 15, at least 20%, at least 25%, at least 30%, at least 35%, at least 40, at least 45%, at least 50%, at least 55%, at least 60, at least 65, at least 70%, at least 75, at least 80%, at least 82%, at least 85%, at least 90%, or at least 95% of the digested precursor dsDNA may be incorporated into partially protected dsDNA. Preferably, the ligation is at least 15% efficient.

The step of ligation of the digested precursor dsDNA to the first and second adaptor molecules may be performed at a second temperature of 1°C -90°C, 2°C -70°C, 5°C-60°C, 8°C-55°C, 9°C-50°C, 10°C-45°C, 11°C-40°C, 12°C-37°C, 13°C-30°C, 14°C-25°C, 15°C-20°C or at about 16°C.

The step of incubating the single contiguous aqueous volume may comprise incubating at a first temperature and then incubating at a second temperature. The first temperature may be 1°C-100°C, 1°C-80°C, 5°C-70°C, 10°C-60°C, 15°C-55°C, 20°C-50°C, 25°C-45°C, 30°C-40°C, 35°C-39°C, 36°C-38°C, or about 37°C. The second temperature may be 1°C -90°C, 2°C -70°C, 5°C-60°C, 8°C-55°C, 9°C-50°C, 10°C-45°C, 11°C-40°C, 12°C-37°C, 13°C-30°C, 14°C-25°C, 15°C-20°C or at about 16°C. Preferably, the first temperature is 35°C-39°C. Preferably, the second temperature is 14°C-18°C.

The step of incubating the single contiguous aqueous volume may be performed isothermally. The step of incubating the single contiguous aqueous volume may comprise incubating at a constant temperature. The constant temperature promotes simultaneous digestion of the double-stranded DNA molecule to produce the linear portion of the double-stranded DNA molecule and ligation of the linear double-stranded region to the first and second adaptor molecules. For example, the constant temperature may be 20°C, 21°C, 22°C, 23°C, 24°C, 25°C, 26°C, 27°C, 28°C, 29°C, 30°C, 31°C, 32°C, 33°C, 34°C, 35°C, 36°C, 37°C, 38°C, 39°C, or 40°C. Preferably, the constant temperature is 30°C. The constant temperature is intended to mean that the temperature does not significantly change during the reaction. The constant temperature is intended to mean that the temperature variation during the step of incubating the single contiguous aqueous volume is less than 10°C, less than 9°C, less than 8°C, less than 7°C, less than 6°C, less than 5°C, less than 4°C, less than 3°C, less than 2°C, or less than 1°C. In a preferred embodiment the temperature during the step of incubating the single contiguous aqueous does not deviate by more than 5°C, preferably by not more than 3°C, even more preferably not more than 1°C. Thus, the constant temperature may be a temperature in a range of 20°C-30°C, 22°C-32°C, 24°C-34°C, 26°C-36°C, 28°C-38°C, 30°C-40°C, 22°C-28°C, 32°C-38°C, 25°C-35°C, 26°C-34°C, 27°C- 33°C, 27.5°C-32.5°C, 28°C-32°C, 28.5°C-31.5°C, 29°C-31°C, or 29.5°C-30.5°C. Preferably, the constant temperature is a temperature in a range of 27.5°C-32.5°C. Alternatively, the constant temperature may be a temperature in a range of 32°C-42°C, 33°C-41°C, 34°C-40°C, 35°C-39°C, 36°C-38°C. Preferably, the constant temperature is a temperature in a range of 34.5°C-39.5°C.

The step of incubating the single contiguous aqueous volume may comprise cycling between the first temperature and the second temperature. The step of incubating the single contiguous aqueous volume may comprise cycling between the first temperature and the second temperature at least 2, at least 3, at least 4, at least 5, at least 6, at least 7, at least 8, at least 9, at least 10, at least 15, at least 20, at least 25, at least 30, at least 35, at least 40, at least 45, at least 50, at least 55, at least 60, at least 65, at least 70, at least 80, at least 90, or at least 100 times, preferably at least 20 times. The step of incubating the single contiguous aqueous volume may comprise cycling between the first temperature and the second temperature less than 40, less than 35, less than 30 times, less than 29, less than 25 times. The step of incubating the single contiguous aqueous volume may comprise cycling between the first temperature and the second temperature 2-100, 5-80, 10-70, 20-60, or 30-60 times. The step of incubating the single contiguous aqueous volume may comprise cycling between the first temperature and the second temperature 2-20, 5-29, 61-100, or 65-80 times.

The DNA template may comprise at least one cleavable target sequence. The cleavable target sequence may be an endonuclease target sequence. Thus, the DNA template may comprise at least one endonuclease target sequence. Preferably, the DNA template comprises at least two endonuclease target sequences. The endonuclease target sequences may be the same or different. Preferably, the at least one endonuclease target sequence is a restriction endonuclease target sequence. Different restriction endonuclease target sequences would be known to the skilled person. The cleavable target sequence may be a Type IIS restriction endonuclease target sequence. For example, the restriction endonuclease target sequence may be a BbsI, BsaI, BsmBI, BspQI, BtgZI, Esp3I,SapI, AarI, Acc36I AcIWI, AcuI, AjuI, AloI, AIw26I, AlwI, ArsI, AsuHPI, BaeI, BarI, BbvI, BccI, BceAI, BcgI, BciVI, BcoDI, BfuAI, BfuI, BmrI, BmsI, BmuI, BpiI, BpmI, BpuEI, BsaXI, Bse1I, Bse3DI, BseGI, BseMI, BseMII, BseNI, BseRI, BseXI, BsgI, BsIFI, BsmAI, BsmFI, BsmI, Bso31I, BspCNI, BspMI, BspPI, BspQI, BspTNI, BsrDI, BsrI, Bst6I, BstF5I, BstMAI, BstV1I, BstV2I, BsuI, BtgZI, BtsCI, BtsI-v2, BtsMutI, BveI, CseI, CspCI, Eam11041, EarI, EciI, Eco31I, Eco57I, Esp3I, FaqI, FauI, FokI, GsuI, HgaI, HphI, HpyAV, LguI, LmnI, Lsp1109I, LweI, MbolI, MlyI, MmeI, MnlI, Mva1269I, NmeAIII, PaqCI, PciSI, PctI, PleI, PpsI, PsrI, SchI, SfaNI, TaqII, TspDTI and/or TspGWI target sequence. The at least one cleavable sequence (e.g. endonuclease target sequence) may be a native cleavable sequence (i.e. a cleavable sequence present in the template molecule). Alternatively, the at least one cleavable sequence (e.g. endonuclease target sequence) may be introduced to the DNA template molecule prior to the production of the protected DNA.

The DNA template used in the methods described herein may be single-stranded or double-stranded. Preferably, the DNA template is double-stranded. The DNA template may be a natural circular DNA molecule. For example, the DNA template molecule may be (i) a plasmid, (ii) a minicircle, (iii) a cosmid, (iv) a bacterial artificial chromosome (BAC), or (v) a molecular inversion probe (MIP). The DNA template molecule may be an enzymatically produced circular DNA molecule. For example, the DNA template may be (i) a circular DNA molecule obtained from recombinase reaction, preferably Cre recombinase reaction, or (ii) a circular DNA molecule obtained from ligase reaction, preferably using the golden gate assembly. The DNA template may be an enzymatically produced covalently-closed linear DNA molecule. For example, the DNA template may be (i) a DNA molecule processed with TelN protelomerase; or (ii) a DNA molecule generated by ligation of the DNA ends with an adaptor. The DNA template molecule may comprise an element that is double-stranded and an element that is single-stranded. For example, the DNA template may comprise a double-stranded DNA and a single-stranded hairpin loop.

The DNA template may be linear. If the DNA template is linear, prior to amplification (e.g. rolling circle amplification), a DNA template may be circularized to produce a DNA template suitable for use in the methods described herein.

The DNA template may comprise a cassette. The cassette may be a mammalian expression cassette. The cassette may further comprise a promoter. The promoter may be a CMV promoter. The cassette may further comprise an enhancer. The cassette may further comprise a reporter gene, such as an eGFP reporter gene or a luciferase reporter gene. The cassette may further comprise a homopolymeric sequence. The cassette may further comprise a LoxP sequence, preferably two LoxP sequences. If the two LoxP sequences are oriented in the same direction, the DNA sequence between the two LoxP sequences is excised as a circular loop of DNA. If the two LoxP sequences are oriented in the opposite direction, the DNA sequence between the two LoxP sequences is inverted. Thus, preferably, the two LoxP sequences are in the same orientation (i.e. the same direction) in the template DNA molecule.

The DNA template may comprise a homopolymeric sequence at a 5'-end or a 3'-end or both a 5'-end and a 3'-end. The homopolymeric sequence may be added to the DNA template molecule before circularization. The homopolymeric sequence may be a polyA, a polyC, a polyG, or a polyT sequence. The homopolymeric sequence may be between 3-200 nucleotides in length. The homopolymeric sequence may be used to facilitate purification of the protected DNA, in which case, the homopolymeric sequence may be between 4-12 nucleotides in length, or between 5-10 nucleotides in length. The homopolymeric sequence may be used to improve mRNA expression, in which case, the homopolymeric sequence may be between 10-200 nucleotides in length, preferably between 80-150 nucleotides in length. The homopolymeric sequence may be at least 10, at least 20, at least 30, at least 40, at least 50, at least 60, at least 70, at least 80, at least 90, at least 100, at least 110, at least 120, at least 130, at least 140, at least 150, at least 160, at least 170, at least 180, at least 190, or at least 200 nucleotides in length. Preferably, the homopolymeric sequence is at least 100 nucleotides in length. More preferably still, the homopolymeric sequence is at least 120 nucleotides in length. For example, the homopolymeric sequence may comprise a polyA sequence of at least 120 nucleotides.

The digested precursor dsDNA may be at least 50, at least 100, at least 250, at least 500, at least 1000, at least 2000, at least 3000, at least 4000, at least 5000, at least 6000, at least 7000, at least 8000, at least 9000, at least 10,000, at least 11,000, at least 12,000, at least 13,000, at least 14,000, or at least 15,000 base pairs long. Preferably, the digested precursor dsDNA is at least 200 base pairs long.

The first end of the digested precursor dsDNA may be complementary to a portion of the first adaptor molecule. The second end of the digested precursor dsDNA may be complementary to a portion of the second adaptor molecule. The first end and/or the second end of the digested precursor dsDNA may be generated by endonuclease digestion.

The digested precursor dsDNA may comprise a 3'-OH group at first and/or second ends. The 3'-OH group may facilitate ligation to the first and/or second adaptor molecule(s) (which may comprise a 5' phosphate). The digested precursor dsDNA may comprise a 5' phosphate at first and/or second ends. The 5' phosphate may facilitate ligation to the first and/or second adaptor molecule(s) (which may comprise a 3'-OH group).

The digested precursor dsDNA may comprise an overhang. For example, the digested precursor dsDNA may comprise a 5' overhang or a 3' overhang. The digested precursor dsDNA may comprise a blunt end or blunt ends. The digested precursor dsDNA may comprise: a 5' overhang and a blunt end, two 5' overhangs, a 3' overhang and a blunt end, two 3' overhangs, or a 5' overhang and a 3' overhang. The overhang may have at least 3 nucleotides (preferably from 4 to 8 nucleotides). The overhang may be in the first or second strand of the digested precursor dsDNA .

### 4. Pharmaceutical compositions and methods for producing pharmaceutical compositions

The invention provides a pharmaceutical composition comprising a protected DNA described herein optionally produced by (or obtainable by) the methods described herein, and a pharmaceutically acceptable carrier or excipient.

The invention provides a method for producing a pharmaceutical composition comprising a protected DNA described herein, wherein the method comprises providing a protected DNA described herein or performing a method described herein to produce a protected DNA and formulating the protected DNA with a pharmaceutically acceptable carrier or excipient.

The invention provides a method for producing a pharmaceutical composition, wherein the method comprises:
(a) providing a protected DNA described herein or producing a protected DNA by a method described herein;
(b) formulating the protected DNA with a pharmaceutically acceptable carrier or excipient.

The pharmaceutical composition may be formulated as pills, tablets or capsules combined with one or more pharmaceutically acceptable solid carriers or as a solution in one or more pharmaceutically acceptable solvents, or as an emulsion, suspension or dispersion in one or more pharmaceutically acceptable solvents or carriers. The formulation may also include other pharmaceutically acceptable excipients such as stabilizers, anti-oxidants, binders, colouring agents or emulsifying or taste-modifying agents and extended release formulations.

The pharmaceutical composition may be administered orally, topically, parenterally or transdermally or by inhalation. The pharmaceutical composition may be administered by injection or intravenous infusion using suitable sterile solutions. Topical dosage forms may be creams, ointments, patches, or similar vehicles suitable for transdermal and topical dosage forms.

The pharmaceutical composition may be dissolved or suspended in a liquid vehicle or formulated as a granule (a small particle or grain), a pellet (a small sterile solid mass consisting of a highly purified composition, with or without excipients, made by the formation of granules, or by compression and moulding), or a pellet coated extended release (a solid dosage form in which the composition itself is in the form of granules to which varying amounts of coating have been applied, and which releases the composition in such a manner to allow a reduction in dosing frequency as compared to that composition presented as a conventional dosage form).

Other forms of pharmaceutical compositions include pills (a small, round solid dosage form containing the composition intended for oral administration), powder (an intimate mixture of dry, finely divided composition with one or more pharmaceutically acceptable additives that may be intended for internal or external use), elixir (a clear, pleasantly flavoured, sweetened hydroalcoholic liquid containing dissolved composition; it is intended for oral use), chewing gum (a sweetened and flavoured insoluble plastic material of various shapes which when chewed, releases the composition into the oral cavity), syrup (an oral solution containing the composition and high concentrations of sucrose or other sugars; the term has also been used to include any other liquid dosage form prepared in a sweet and viscid vehicle, including oral suspensions), tablet (a solid dosage form containing the composition with or without suitable diluents), tablet chewable (a solid dosage form containing the composition with or without suitable diluents that is intended to be chewed, producing a pleasant tasting residue in the oral cavity that is easily swallowed and does not leave a bitter or unpleasant after-taste), tablet coated or tablet delayed release, tablet dispersible, tablet effervescent, tablet extended release, tablet film coated, or tablet film coated extended release where the tablet is formulated in such manner as to make the contained composition available over an extended period of time following ingestion.

In other forms of pharmaceutical compositions, a tablet for solution, tablet for suspension, tablet multilayer, tablet multilayer extended release may be provided, where the tablet is formulated in such manner as to allow at least a reduction in dosing frequency as compared to that composition presented as a conventional dosage form. A tablet orally disintegrating, tablet orally disintegrating delayed release, tablet soluble, tablet sugar coated, osmotic, and the like are also suitable.

The oral dosage form pharmaceutical composition may contain, in addition to the composition, one or more inactive pharmaceutical ingredients such as diluents, solubilizers, alcohols, binders, controlled release polymers, enteric polymers, disintegrants, excipients, colorants, flavorants, sweeteners, antioxidants, preservatives, pigments, additives, fillers, suspension agents, surfactants (e.g., anionic, cationic, amphoteric and nonionic), and the like. Various FDA-approved topical inactive ingredients are found at the FDA's "The Inactive Ingredients Database" that contains inactive ingredients specifically intended as such by the manufacturer.

As used herein, injectable and infusion dosage forms include, but are not limited to, a liposomal injectable, which either consists of or forms liposomes (a lipid bilayer vesicle usually composed of phospholipids which is used to encapsulate the composition); an injection, which includes a sterile preparation intended for parenteral use; an emulsion injection, which includes an emulsion consisting of a sterile, pyrogen-free preparation intended to be administered parenterally; or a lipid complex injection.

For example, the protected DNA can be administered by intratympanic injection (e.g. into the middle ear) and/or injections into the outer, middle, and/or inner ear. Such methods are routinely used in the art, for example, for the administration of steroids and antibiotics into human ears. Injection can be, for example, through the round window of the ear or through the cochlear capsule.

Other forms of pharmaceutical composition include a powder for solution injection, which is a sterile preparation intended for reconstitution to form a solution for parenteral use; a powder for suspension injection that is a sterile preparation intended for reconstitution to form a suspension for parenteral use; a powder lyophilized for liposomal suspension injection, which is a sterile freeze dried preparation intended for reconstitution for parenteral use which has been formulated in a manner that would allow liposomes (a lipid bilayer vesicle usually composed of phospholipids which is used to encapsulate the composition, either within a lipid bilayer or in an aqueous space) to be formed upon reconstitution; or a powder lyophilized for solution injection, wherein lyophilization ("freeze drying") is a process which involves the removal of water from products in the frozen state at extremely low pressures.

A suspension injection comprises a liquid preparation, suitable for injection, which consists of solid particles dispersed throughout a liquid phase in which the particles are not soluble that can also consist of an oil phase dispersed throughout an aqueous phase, or vice-versa. A suspension liposomal injection comprises a liquid preparation, suitable for injection, which consists of an oil phase dispersed throughout an aqueous phase in such a mannerthat liposomes (a lipid bilayer vesicle usually composed of phospholipids which is used to encapsulate the composition, either within a lipid bilayer or in an aqueous space) are formed. A suspension sonicated injection comprises a liquid preparation, suitable for injection, which consists of solid particles dispersed throughout a liquid phase in which the particles are not soluble. In addition, the product is sonicated while a gas is bubbled through the suspension, and this results in the formation of microspheres by the solid particles.

In another mode of administration, the pharmaceutical composition can be administered in situ, via a catheter or pump. A catheter or pump can, for example, direct the composition into the target location.

The parenteral carrier system may include one or more pharmaceutically suitable excipients, such as solvents and co-solvents, solubilizing agents, wetting agents, suspending agents, thickening agents, emulsifying agents, chelating agents, buffers, pH adjusters, antioxidants, reducing agents, antimicrobial preservatives, bulking agents, protectants, tonicity adjusters, and special additives. Formulations suitable for parenteral administration conveniently comprise a sterile oily or aqueous preparation of the composition which is preferably isotonic with the blood of the recipient but this is not essential.

As used herein, inhalation dosage forms include, but are not limited to, an aerosol (a product that is packaged under pressure and contains the composition which is released upon activation of an appropriate valve system intended for topical application to the skin as well as local application into the nose (nasal aerosols), mouth (lingual and sublingual aerosols), or lungs (inhalation aerosols)). A foam aerosol is a dosage form containing the composition, surfactants, aqueous or nonaqueous liquids, and propellants, whereby if the propellant is in the internal (discontinuous) phase (i.e., of the oil-in-water type), a stable foam is discharged, and if the propellant is in the external (continuous) phase (i.e., of the water-in-oil type), a spray or a quick-breaking foam is discharged. A metered aerosol is a pressurized dosage form consisting of metered dose valves which allow for the delivery of a uniform quantity of spray upon each activation. A powder aerosol is a product that is packaged under pressure and contains the composition, in the form of a powder that is released upon activation of an appropriate valve system. An aerosol spray is an aerosol product which utilizes a compressed gas as the propellant to provide the force necessary to expel the product as a wet spray and being applicable to solutions of the composition in aqueous solvent(s).

A transdermal dosage form may include, but is not limited to, a patch (a drug delivery system that often contains an adhesive backing that is usually applied to an external site on the body, whereby the ingredients (including the composition) either passively diffuse from, or are actively transported from, some portion of the patch, and whereby depending upon the patch, the ingredients (including the composition are either delivered to the outer surface of the body or into the body. Various types of transdermal patches such as matrix, reservoir and others are known in the art.

A topical dosage form may include various dosage forms known in the art such as lotions (an emulsion, liquid dosage form, whereby this dosage form is generally for external application to the skin), lotion augmented (a lotion dosage form that enhances composition delivery, whereby augmentation does not refer to the strength of the composition in the dosage form), gels (a semisolid dosage form that contains a gelling composition to provide stiffness to a solution or a colloidal dispersion, whereby the gel may contain suspended particles) and ointments (a semisolid dosage form, usually containing less than 20% water and volatiles and greater than 50% hydrocarbons, waxes, or polyols as the vehicle, whereby this dosage form is generally for external application to the skin or mucous membranes). Further embodiments include ointment augmented (an ointment dosage form that enhances composition delivery, whereby augmentation does not refer to the strength of the composition in the dosage form), creams (an emulsion, semisolid dosage form, usually containing greater than 20% water and volatiles and/or less than 50% hydrocarbons, waxes, or polyols may also be used as the vehicle, whereby this dosage form is generally for external application to the skin or mucous membranes) and cream augmented (a cream dosage form that enhances composition delivery, whereby augmentation does not refer to the strength of the composition in the dosage form). As used herein, an "emulsion" refers to a dosage form consisting of a two-phase system comprised of at least two immiscible liquids, one of which is dispersed as droplets, internal or dispersed phase, within the other liquid, external or continuous phase, generally stabilized with one or more emulsifying agents, whereby emulsion is used as a dosage form term unless a more specific term is applicable (e.g. cream, lotion, ointment). Further embodiments include suspensions (a liquid dosage form that contains solid particles dispersed in a liquid vehicle), suspension extended release, pastes (a semisolid dosage form, containing a large proportion, 20-50%, of solids finely dispersed in a fatty vehicle, whereby this dosage form is generally for external application to the skin or mucous membranes), solutions (a clear, homogeneous liquid dosage form that contains one or more chemical substances dissolved in a solvent or mixture of mutually miscible solvents), and powders.

The topical dosage form composition contains the composition and one or more inactive pharmaceutical ingredients such as excipients, colorants, pigments, additives, fillers, emollients, surfactants (e.g., anionic, cationic, amphoteric and nonionic), penetration enhancers (e.g., alcohols, fatty alcohols, fatty acids, fatty acid esters and polyols), and the like. Various FDA-approved topical inactive ingredients are found at the FDA's "The Inactive Ingredients Database" that contains inactive ingredients specifically intended as such by the manufacturer.

### 5. Applications of protected DNA

The invention provides a method for *in vitro* transcription of a protected DNA, wherein the method comprises contacting the protected DNA of the invention, with a polymerase and producing a transcription product encoded by the protected DNA.

The invention provides a method for *in vitro* transcription of a protected DNA, wherein the method comprises:
(a) providing a protected DNA as described herein or producing a protected DNA by a method described herein; and
(c) producing a transcription product encoded by the protected DNA.

The invention provides a method for *in vitro* transcription of a protected DNA, wherein the method comprises:
(a) providing a protected DNA as described herein or producing a protected DNA by a method described herein;
(b) contacting the protected DNA with a polymerase; and
(c) producing a transcription product encoded by the protected DNA.

The invention provides a method for *in vitro* transcription of a protected DNA, wherein the method comprises:
(a) providing a protected DNA as described herein or producing a protected DNA by a method described herein and hybridising a single-stranded DNA to the ssDNA cassette to provide a double-stranded promoter for a polymerase (e.g. T7 polymerase);
(b) contacting the protected DNA with a polymerase (e.g. T7 polymerase); and
(c) producing a transcription product encoded by the protected DNA.

The invention provides a method for producing a protein, wherein the method comprises introducing the protected DNA of the invention into a cell (e.g. a prokaryotic cell or a eukaryotic cell) or a cell-free expression system to generate a protein encoded by the protected DNA.

The invention provides a method for producing a protein, wherein the method comprises:
(a) providing a protected DNA as described herein or producing a protected DNA by a method described herein; and
(b) introducing the protected DNA into a cell (e.g. a prokaryotic cell or a eukaryotic cell) or a cell-free expression system to generate a protein encoded by the protected DNA.

The cell-free expression system may originate from (or be derived from) a prokaryotic cell or eukaryotic cell. For example, the cell-free expression system may originate from (or be derived from) rabbit reticulocytes, wheat germ or *Escherichia coli.*

The cell may be a prokaryotic cell or a eukaryotic cell. The cell may be an animal cell, such as mammal cell (e.g. a human cell), a fungal cell, a cell of a micro-organism (e.g. a prokaryotic cell or a eukaryotic cell), or a plant cell. Preferably, the cell is a human cell.

The protected DNA may comprise a cassette. The desired protein may be encoded by the cassette.

The step of introducing the protected DNA into a cell may be performed *in vivo* or *in vitro.*

The nuclease-resistant nucleotides (i.e. protected nucleotides) may be any protected nucleotides described herein.

The invention provides a method for cell transfection of a protected DNA described herein into a cell.

The invention provides a method for cell transfection of a protected DNA described herein into a cell, wherein the method comprises:
(a) providing a protected DNA described herein optionally produced (or obtainable) by the methods described herein;
(b) contacting a cell with the protected DNA; and
(c) transfecting the protected DNA into the cytosol of the cell.

The invention provides a cell transfection composition comprising a protected DNA described herein optionally produced by (or obtainable by) the methods described herein.

The cell transfection composition may or may not comprise a carrier e.g. an agent or a formulation. Preferably, the carrier promotes accumulation of the protected DNA at a target site and/or protects the protected DNA from undesirable interactions with biological milieu components and/or protects the protected DNA from metabolism and/or degradation.

The invention further provides a cell transfection method comprising contacting (*in vitro*) a cell to be transfected with a protected DNA described herein, and wherein the protected DNA is transfected into the cytosol of the cell.

The cell or cells to be transfected may be provided in a cell culture medium (e.g. in a Petri dish, culture vessel or well, etc). The protected DNA may be added directly to the cell culture medium or the cells may be added to a solution, such as saline, a buffered solution or a cell culture medium, comprising the protected DNA.

The carrier may be a viral carrier or a non-viral carrier. Viral carriers include a lentiviral vector, an adenoviral vector, a retroviral vector, or an adeno associate viral vector for delivery of the protected DNA . Non-viral carriers (or vectors) include complexing the protected DNA with a cationic agent such as a cationic cell penetrating peptide (CPP); a DNA-binding cationic component, such as a polylysine chain; a cationic polymer or dendrimer e.g. polyethylenimine (PEI), poly-D,L-lactide-co-glycolide (PLGA), and a block copolymer of PEG and polylysine, and/or a cationic lipid (e.g. lipofectamine).

The carrier may be a small molecule (e.g., cholesterol, bile acid, and/or lipid), polymer, protein (e.g. an antibody), and/or aptamer (e.g. RNA) that is conjugated to the protected DNA . The carrier may be a nanoparticulate formulation used to encapsulate the protected DNA .

The carrier may be a modification of the protected DNA with a targeting ligand (e.g. an antibody), a peptide, a small molecule (e.g. folic acid and/or biotin), or a polymer present in extracellular matrix (e.g. hyaluronic acid and/or chondroitin sulphate), or a hydrophobic modification (e.g. using cholesterol and/or α-tocopherol).

The cell transfection composition may or may not comprise an agent selected from a photosensitizing agent and/or a radical initiator e.g. a photoinitiator. Preferably, this agent improves the function of the protected DNA at a target site and/or protects the protected DNA from metabolism and/or degradation.

The invention further provides a cell obtainable by the methods of the invention. Thus, the cell may comprise a protected DNA described herein optionally produced by (or obtainable by) the methods described herein.

The invention further provides a cell transfected with a protected DNA described herein optionally produced by (or obtainable by) the methods described herein.

The cell may be an animal cell, such as mammal cell (e.g. a human cell), a fungal cell, a cell of a micro-organism (e.g. a prokaryotic cell or a eukaryotic cell), or a plant cell. Preferably, the cell is a human cell.

The step of contacting the cell with a protected DNA may be performed *in vivo.* For example the protected DNA may be administered to an organism (e.g. a subject) in need thereof. The organism (e.g. the subject) may be an animal, such as mammal (e.g. a human), a fungus, a micro-organism, or a plant.

Any or all of the protected DNA described herein may be delivered to a cell via delivery particles such as liposomes, nanoparticles, exosomes, macrovesicles, viral or non-viral vectors. Any or all of the protected DNA described herein may be delivered to a cell using a gene-gun. Any or all of the protected DNA described herein may be delivered to a cell by electroporation. Any or all of the protected DNA described herein may be delivered to a cell by hydrodynamic needle. The protected DNA described herein may be delivered to a cell without a carrier.

The invention provides a use of a protected DNA as described herein in the production of viral or non-viral delivery system.

The invention provides a use of a protected DNA in the production of viral or non-viral delivery system, wherein the protected DNA is as described herein or produced by performing a method described herein.

The invention provides a viral or non-viral delivery system comprising a protected DNA as described herein. The invention provides a viral or non-viral delivery system comprising a protected DNA, wherein the protected DNA is as described herein or produced by performing a method described herein.

### Viral vectors

Methods of producing viral vectors, such as AAV vectors, are known in the art. The most widely used method involves the co-transfection of HEK293 by three bacterial plasmids. The first plasmid encodes the Rep and Cap element, the second plasmid is a helper plasmid, while the third plasmid encodes the genetic payload of interest with inverted terminal repeats (ITRs). There are several issues surrounding the use of plasmids for viral preparation (e.g. AAV), namely: 1) the production of the plasmids is time consuming and costly, 2) there is a difficulty in the propagation of ITR sequences in *E.coli*; 3) the incorporation of the plasmid backbone into the viral capsid (e.g. AAV capsid) might be challenging (e.g. issues with antibiotic resistance markers). Methods of producing viral vectors may be using other cell lines. For example, a cell line suitable for production of viral vectors may be a Vero cell, or any other stable cell line. Together these represent the main bottleneck in viral vector production (e.g. AAV production). Thus, the methods described herein provide a protected DNA suitable for use in production of viral vectors. The protected DNA overcomes the above issues with plasmid vectors.

The invention provides a method of producing a viral vector, the method comprising introducing the protected DNA as described herein or as produced by a method described herein into a cell under conditions such that the viral vector is produced. The protected DNA may encode at least one element required for the production of the viral vector. For example, the protected DNA may encode Rep and/or Cap elements. The protected DNA may encode the helper plasmid elements. The protected DNA may encode Rep, Cap and helper plasmid elements. The protected DNA may encode a transgene. The method may be an in vivo or in vitro method. The cell may be an animal cell, preferably mammal cell, such as human cell (e.g. HEK293T, HEK293, CAP, CAP-T, or CHO). The cell may be a cell cultured in vitro in a tissue culture cell line.

Preferably, the vector is an AAV vector or lentivirus vector.

The invention also provides a method of delivering the viral vector (e.g. protected DNA) to a cell, comprising contacting the viral vector as described herein or produced by a method described herein with the cell. The cell may be an animal cell, preferably mammal cell, such as human cell.

The invention also provides a cell obtainable by the methods described herein.

### Non-viral vectors

Methods of producing non-viral vectors, are known in the art. Use of non-viral vectors over viral vectors has several advantages, including the opportunity of repeat dosing due to their non-immunogenicity, an unlimited packaging capacity, and low associated toxicity. Most methods for producing non-viral vectors use plasmid DNA. Thus, the protected DNA described herein or produced by a method described herein is suitable for use in production of non-viral vectors. The protected DNA described herein or produced by a method described herein overcomes the issues of using plasmid vectors in non-viral vector preparation. For example, the protected DNA, unlike plasmid DNA, does not comprise a bacterial backbone, allowing more transgene copies per mg of DNA. In addition, the protected DNA does not comprise antibiotic resistant genes and bacterial contaminants. Moreover, the protected DNA provides a prolonged transgene expression (due to the presence of exonuclease-resistant nucleotides), and a more cost-effective process of non-viral vector production.

The invention provides a method of delivering the non-viral vector to a cell, comprising contacting the non-viral vector comprising the protected DNA with the cell. The cell may be an animal cell, preferably mammal cell, such as human cell.

The invention also provides a cell obtainable by the methods described herein.

### General therapeutic and diagnostic uses

The protected DNA described herein has particular utility as a therapeutic agent (i.e. DNA therapeutic) which can be used to express a gene product *in vivo.* This is because its protected structure (e.g. the presence of nuclease-resistant nucleotides) prevents attack by enzymes such as exonucleases, leading to enhanced stability and longevity of gene expression as compared to DNA molecules with unprotected ends. Unprotected cassettes have been demonstrated to be inefficient with respect to gene expression when introduced into host tissue. This has been attributed to cassette instability due to the action of exonucleases in the extracellular space.

The present invention may be used for the production of DNA for *in vitro* expression in a host cell, for example, in DNA vaccines. DNA vaccines typically encode a modified form of an infectious organism's DNA. DNA vaccines are administered to a subject where they then express the selected protein of the infectious organism, initiating an immune response against that protein which is typically protective. DNA vaccines may also encode a tumour antigen in a cancer immunotherapy approach.

Other types of therapeutic DNA molecules are also contemplated e.g. those used in gene therapy. For example, such DNA molecules can be used to express a functional gene where a subject has a genetic disorder caused by a dysfunctional version of that gene. Examples of such diseases include sickle cell anaemia, cystic fibrosis, Huntington disease, Duchenne's Muscular Dystrophy, Haemophilia A, α1-antitrypsin deficiency, primary ciliary dyskinesia, or respiratory distress syndrome of prematurity. Other diseases where gene therapy may be useful include metabolic diseases, respiratory diseases, inflammatory diseases, autoimmune, chronic and infectious diseases, including such disorders as AIDS, cancer, neurological diseases, cardiovascular disease, hypercholesterolemia, various blood disorders including various anaemias, thalassemia and haemophilia, and emphysema. For the treatment of solid tumours, genes encoding toxic peptides (i.e., chemotherapeutic agents such as ricin, diptheria toxin and cobra venom factor), tumour suppressor genes such as p53, genes coding for mRNA sequences which are antisense to transforming oncogenes, antineoplastic peptides such as tumor necrosis factor (TNF) and other cytokines, or transdominant negative mutants of transforming oncogenes, may be expressed.

The protected DNA described herein is particularly suitable for use in therapy. The invention provides a protected DNA as described herein for use in therapy. The invention provides a protected DNA obtainable by the method described herein for use in therapy. The protected DNA may encode a sequence of a therapeutic protein, a part of a vaccine, or an element of a genetic engineering mechanism, which is used to treat a disease or infection in a subject.

The invention further provides the protected DNA as described herein for use as a medicament. The invention further provides the protected DNA obtainable by the methods described herein for use as a medicament. The invention also provides the use of a protected DNA as described herein for the manufacture of a medicament for treating a disease. The invention also provides the use of a protected DNA obtainable by the methods described herein for the manufacture of a medicament for treating a disease.

The protected DNA may encode a sequence of a therapeutic protein, a part of a vaccine, or an element of a genetic engineering mechanism, which is used to treat a disease or infection in a subject.

The invention further provides the protected DNA produced by the methods described herein for use in treating a disease.

The invention also provides a method of treating a disease in a subject comprising administering to the subject a protected DNA described herein optionally produced (or obtainable) by a method described herein. The invention also provides a method of treating a disease in a subject comprising administering to the subject the protected DNA obtainable by the methods described herein. Preferably, the amount of the protected DNA administered to the subject is a therapeutic active amount.

The protected DNA described herein may be used to treat any disease or disorder. For example, the protected DNA may be used to treat one or more of the diseases and/or disorders selected from genetic disorders (e.g. monogenic disorders), cancer, HIV, other viral infections (e.g. infection caused by coronavirus (e.g. COVID-19), hepatitis A, hepatitis B, herpes simplex virus type 2, influenza, measles and/or respiratory syncytial virus), neurodegenerative diseases (e.g. Parkinson's disease and/or polyglutamine diseases such as Huntington's disease), ocular diseases (e.g. macular degeneration) and liver failure. Preferably, the protected DNA is used to treat a genetic disorder. More preferably still, the protected DNA is used to treat a monogenic disorder. For example, the protected DNA, may be used to treat sickle cell anaemia, cystic fibrosis, Huntington disease, and Duchenne's Muscular Dystrophy, Haemophilia A, α1-antitrypsin deficiency, primary ciliary dyskinesia, or respiratory distress syndrome of prematurity.

A subject treated with the protected DNA may receive the protected DNA in the form of any of the pharmaceutical compositions described herein.

A subject treated with the protected DNA may receive the protected DNA in combination with other forms of treatment for the disorder concerned, including treatment with drugs generally used for the treatment of the disorder. The drugs may be administered in one or several dosage units. The skilled person (e.g. a medical practitioner) is well able to determine an appropriate dosage regimen for the subject according to the subject's specific circumstances.

As used herein, "administering" means introducing the protected DNA into the subject's body as described in more detail above (see "Methods for producing a pharmaceutical composition"). Examples include but are not limited to oral, topical, buccal, sublingual, pulmonary, transdermal, transmucosal, as well as subcutaneous, intraperitoneal, intravenous, and intramuscular injection or in the form of liquid or solid doses via the alimentary canal.

As used herein, the phrase "a therapeutically active amount" means an amount of the protected DNA that, when administered to a subject for treating a disease, is sufficient to effect such treatment of the disease. A "therapeutically active amount" will vary depending, for instance, on factors such as the specific product used, the severity of subject's disease, the age and relative health of the subject and the route and form of administration. Determining the relevant therapeutically active amount for a specific subject based on such factors is routine for the person skilled in the art (e.g. an attending medical practitioner). Treatment of a disease as described herein should be understood to mean an improvement in one of more of the symptoms of a disease.

The invention also provides the use of the protected DNA as described herein in a method of diagnosing a disease and/or a disorder. The invention also provides the use of the protected DNA obtainable by the methods described herein in a method of diagnosing a disease and/or a disorder.

The invention provides the use of the protected DNA in the "in vitro" diagnosis of a disease. The invention provides the use of the protected DNA obtainable by the methods described herein in the "in vitro" diagnosis of a disease.

The invention also provides the protected DNA for use in a method of diagnosis "in vivo" of a disease.

The method may be used to diagnose any disease and/or disorder. For example, the diseases and/or disorders may be selected from genetic disorders (e.g. monogenic disorders), cancer, HIV, other viral infections (e.g. infection caused by coronavirus (e.g. COVID-19), hepatitis A, hepatitis B, herpes simplex virus type 2, influenza, measles and/or respiratory syncytial virus), neurodegenerative diseases (e.g. Parkinson's disease and/or polyglutamine diseases such as Huntington's disease), ocular diseases (e.g. macular degeneration) and liver failure. Preferably, the protected DNA is used to diagnose a genetic disorder. More preferably still, the protected DNA is used to diagnose a monogenic disorder. For example, the protected DNA may be used to diagnose sickle cell anaemia, cystic fibrosis, Huntington disease, and Duchenne's Muscular Dystrophy, Haemophilia A, α1-antitrypsin deficiency, primary ciliary dyskinesia, or respiratory distress syndrome of prematurity.

The diagnostic and treatment methods described herein may be in vitro methods or in vivo methods.

The method of diagnosis may rely on the detection and/or quantification of the protected DNA.

To facilitate detection and/or quantification of the protected DNA, the protected DNA may be attached or bound to a functional portion. The functional portion may be any functional portion described herein. For example the functional portion may be a probe. The functional portion may comprise a fluorophore, a radioactive compound or a barcode. The functional portion may be a protein, for example, an antibody.

The diagnosis may rely on the detection of a signal corresponding to the presence, absence and/or level of the protected DNA. For example, the signal may be measured by flow cytometry and/or fluorescence-activated cell sorting of a protected DNA attached to a fluorescent probe.

The protected DNA may be detected by being bound to a capture moiety, for example in a lateral flow assay. In this example, the functional portion is a protein, for example, an antibody specific for the capture moiety. The capture of the antibody attached to the protected DNA may produce a visual signal (e.g. a band of a different colour).

The invention also provides a method that combines diagnosis of a disease with a treatment of the disease.

### Cell therapy

The products produced by the methods of the invention may be substantially less contaminated than plasmid DNA. In addition, the products produced by the methods of the invention are very simple in nature (e.g. no bacterial backbone), which means that they are typically easy to work with. The pure and simple nature of the products described herein makes them particularly suitable for use in cell therapy. For example, a cell comprising the protected DNA may be injected or otherwise transplanted into a patient to cause a desired effect. The cell (or cells) may be capable of fighting cancer cells, for example, via cell-mediated immunity in the course of immunotherapy. The cell (or cells) may be grafted to regenerate diseased tissues.

The invention provides the protected DNA described herein for use in cell therapy. The invention provides the protected DNA obtainable by the methods described herein for use in cell therapy.

The invention provides the protected DNA described herein for use in cell therapy. The invention provides the protected DNA obtainable by the methods described herein for use in cell therapy.

Preferably, cell therapy is ex-vivo cell therapy. The cell may be an animal cell, preferably mammal cell, such as human cell.

The invention also provides a cell obtainable by any methods described herein. For example, the cell may be suitable for use in cell therapy.

### Vaccines

The protected DNA produced by the methods described herein are particularly suitable for use in vaccine production. A vaccine may comprise a protected DNA described herein. A vaccine may comprise a protected DNA obtainable by the methods described herein. Alternatively, the protected DNA may be used to produce a vaccine, preferably an mRNA-based vaccine. For example, vaccines such as the BioNTech and Moderna mRNA vaccines against COVID-19.

Thus, the invention provides the use of the protected DNA described herein in the production of a vaccine. The invention also provides the use of the protected DNA obtainable by the methods described herein in the production of a vaccine.

The protected DNA may encode an antigen, which may cause an immune response in a subject. The subject may be human. Preferably, the antigen is encoded on the cassette.

### CAR-T cells

The invention provides the use of a protected DNA described herein in the production of a CAR-T cell. The invention provides the use of the protected DNA obtainable by the methods described herein in the production of a CAR-T cell.

The invention provides a method for producing a genetically engineered CAR-T cell comprising: (a) introducing the protected DNA described herein into a T cell; and (b) expressing a gene of interest encoded by the protected DNA. Preferably, the gene of interest is a tumour-specific CAR.

The invention provides a method for producing a genetically engineered CAR-T cell comprising: (a) introducing the protected DNA obtainable by the methods described herein into a T cell; and (b) expressing a gene of interest encoded by the protected DNA. Preferably, the gene of interest is a tumour-specific CAR.

The method may further comprise, before step (a), a step of removing mononuclear cells from a patient. Preferably, the step of removing is performed using leukapheresis. Preferably the mononuclear cells are T cells. The method may further comprise, after step (b), a step of returning the genetically engineered cells to the patient.

The invention also provides an engineered T cell obtainable by any methods described herein. The engineered T cell may be suitable for use in CAR T-cell therapy.

### Gene editing

The protected DNA vector described herein or a protected DNA vector produced (or obtainable) by the methods described herein may be used in gene editing. The gene editing may use a CRISPR associated (Cas) nuclease, a Transcription activator-like effector nuclease (TALEN) and/or a Zinc finger nuclease (ZFN). Preferably, the gene editing may use a CRISPR associated (Cas) nuclease

The protected DNA described herein is particularly suitable for use in delivery of CRISPR, TALEN or ZFN machinery to cells, for example in cell therapy or *in vivo* therapy.

Various different cargos and delivery vehicles are used commonly for delivery of CRISPR machinery, including physical delivery methods (e.g. microinjection; electroporation), viral delivery methods (e.g. adeno-associated virus (AAV); full-sized adenovirus and lentivirus), and non-viral delivery methods (e.g. liposomes; polyplexes; gold particles).

The protected DNA product may comprise a gene sequence encoding any component of the CRISPR, TALEN or ZFN machinery. The protected DNA product may encode all components of the CRISPR, TALEN or ZFN machinery.

The cassette of the protected DNA may comprise (or encode) a repair template (or editing template). The repair template (or editing template) may be for editing genomes, for example, using the CRISPR-Cas system. The repair template (or editing template) may comprise or consist of a homology region (e.g. homology arm) which is homologous to the desired DNA region (i.e. target molecule). The repair template (or editing template) may be for use in CRISPR-Cas mediated homology directed repair (HDR). The repair template (or editing template) may be used to repair a target molecule having a strand break (e.g. a single stranded break or a double stranded break). The strand break may be created by a nuclease of the CRISPR (e.g. Cas9, Cpf1, or MAD7), TALEN or ZFN system. The repair template (or editing template) may introduce at least one mutation (e.g. an insertion, deletion, and/or substitution) in the desired DNA region (i.e. target molecule). The repair template (or editing template) may comprise at least 10, 20, 30, 40, 50, 100, 200, 300, 400, 500, 600, 700, 800, 900, 1000, 1500, 2000, 2500, 3000, 3500, 4000, 4500, 5000, 5500, 6000, 6500, 7000, 7500, 8000, 8500, 9000, 9500, or 10000 base pairs. Preferably, the repair template (or editing template) comprises at least 200 base pairs. The protected DNA encoding the repair template may be delivered to a cell by a nanoparticle, a non-viral vector or a viral vector, or without the aid of any carrier. The protected DNA encoding the repair template may be delivered to a cell by electroporation. The protected DNA encoding the repair template may be delivered to a cell by hydrodynamic needle.

The cassette of the protected DNA may comprise (or further comprise) a gene sequence encoding a nuclease protein of a CRISPR, TALEN and/or ZFN system.

The cassette of the protected DNA may comprise (or further comprise) a gene sequence encoding a nuclease protein of a CRISPR system (e.g. Cas9, Cpf1, or MAD7), and/or a guide RNA. The cassette of the protected DNA may comprise (or further comprise) a gene sequence encoding a nuclease protein of a CRISPR system (e.g. Cas9, Cpf1, or MAD7). The cassette of the protected DNA may comprise (or further comprise) a gene sequence encoding a guide RNA. The cassette of the protected DNA may comprise (or further comprise) a gene sequence encoding a nuclease protein of a CRISPR system (e.g. Cas9, Cpf1, or MAD7) and a guide RNA. The cassette of the protected DNA may comprise (or further comprise) a gene sequence encoding a genomic target to be modified (e.g. a spacer). The cassette of the protected DNA may be ligated to a vector. The vector may comprise a sequence of some of the components of the CRISPR, TALEN and/or ZFN system. The vector may comprise a sequence of guide RNA or a sequence of a part of the guide RNA. If the vector comprises a sequence of a part of the guide RNA, the protected DNA may comprise the missing sequence part of the guide RNA, so that upon ligation, the ligated vector comprises a full sequence of a guide RNA. The nuclease of the CRISPR system and guide RNA may be encoded on a single vector or on two different vectors. The cassette of the protected DNA may encode the nuclease of the CRISPR system and guide RNA. One protected DNA may encode the nuclease of the CRISPR system and another protected DNA may encode guide RNA.

The protected DNA may be for use in CRISPR-Cas, TALEN and/or ZFN mediated repair by recombination, homology-directed repair, or non-homologous end joining.

If the nuclease of the CRISPR system and the guide RNA are encoded by a different protected DNA, they may be part of a different or the same delivery mechanism. For example, the protected DNA encoding the nuclease of the CRISPR system may be delivered to a cell by a first nanoparticle, non-viral vector or viral vector, whereas the protected DNA encoding the guide RNA may be delivered to a cell by a second nanoparticle, a non-viral vector, or viral vector. For example, the protected DNA encoding the nuclease of the CRISPR system and the protected DNA encoding the guide RNA may be delivered to a cell by the same nanoparticle, non-viral vector or viral vector.

If the nuclease of the CRISPR system and the guide RNA are encoded by the same protected DNA (or by a vector that comprises the protected DNA) they may be part of the same delivery mechanism. For example, the protected DNA, or the vector, encoding the nuclease of the CRISPR system and the guide RNA may be delivered to a cell by a nanoparticle, a non-viral vector or a viral vector.

The protected DNA encoding the nuclease of the CRISPR system and the protected DNA encoding the guide RNA may be delivered to a cell by electroporation. The protected DNA encoding the nuclease of the CRISPR system and the protected DNA encoding the guide RNA may be delivered to a cell by hydrodynamic needle.

Thus, the invention provides the protected DNA described herein for use in CRISPR, TALEN and/or ZFN system delivery to a cell. The invention provides a method of delivering the CRISPR, TALEN and/or ZFN system to a cell, comprising contacting the protected DNA described herein with a cell. The invention also provides the protected DNA obtainable by the methods described herein for use in CRISPR, TALEN and/or ZFN system delivery to a cell. The invention provides a method of delivering the CRISPR, TALEN and/or ZFN system to a cell, comprising contacting the protected DNA obtainable by the methods described herein with a cell.

The cell may be an animal cell, preferably mammal cell, such as human cell.

The invention also provides a cell obtainable by the methods described herein. The cell is particularly suitable for use in cell therapy and/or in vivo therapy.

The protected DNA described herein may be used in transcription, to generate RNA, preferably mRNA, *in vitro* or *in vivo.*

### 6. Kits

The invention provides a kit comprising components required to carry out the methods described herein. The kit may comprise at least:
(a) first and second adaptor molecules, wherein each first and second adaptor molecule comprises dsDNA comprising a first strand and a second strand, wherein the first strand of the first adaptor molecule comprises x nuclease-resistant nucleotides and the first strand of the second adaptor molecule comprises y nuclease-resistant nucleotides, wherein x is at least 1 and y is at least 1;
(b) an endonuclease;
(c) a ligase; and
(d) an exonuclease.

The kit may additionally comprise a DNA polymerase, at least one buffer and/or a nuclease.

The first strand of the first adaptor molecule may comprise the sequence of SEQ ID NO: 7 or a portion thereof. The second strand of the first adaptor molecule may comprise the sequence of SEQ ID NO: 8 or 9 or a portion thereof. The first strand of the second adaptor molecule may comprise the sequence of SEQ ID NO: 10 or a portion thereof. The second strand of the second adaptor molecule may comprise the sequence of SEQ ID NO: 11 or 12 or a portion thereof. The first and second adapter molecules may comprise a different nucleic acid sequence.

The first adaptor molecule and/or the second adaptor molecule may comprise one or more locked nucleic acids (LNAs).

The first adaptor molecule and/or the second adaptor molecule may comprise one or more protected nucleotides (i.e. nuclease-resistant nucleotides), such as phosphorothioated nucleotides.

The endonuclease may be a restriction enzyme endonuclease. The endonuclease may be a Type IIS restriction enzyme. The endonuclease may be any enzyme that recognizes a DNA sequence and cleaves outside of the recognition sequence. For example, the endonuclease may be a Bbsl, Bsal, BsmBI, BspQI, BtgZI, Esp3I,SapI, Aarl, Acc36I, AcIWI, Acul, Ajul, Alol, Alw26I, Alwl, Arsl, AsuHPI, Bael, Barl, Bbvl, Bccl, BceAI, Bcgl, BciVI, BcoDI, BfuAI, Bful, Bmrl, Bmsl, Bmul, Bpil, Bpml, BpuEI, BsaXI, Bse1I, Bse3DI, BseGI, BseMI, BseMII, BseNI, BseRI, BseXI, Bsgl, BslFI, BsmAI, BsmFI, Bsml, Bso311, BspCNI, BspMI, BspPI, BspQI, BspTNI, BsrDI, Bsrl, Bst6I, BstF5I, BstMAI, BstV1I, BstV2I, Bsul, BtgZI, BtsCI, Btsl-v2, BtsMutl, Bvel, Csel, CspCI, Eam1104I, Earl, Ecil, Eco31I, Eco57I, Esp3I, Faql, Faul, Fokl, Gsul, Hgal, Hphl, HpyAV, Lgul, Lmnl, Lsp11091, LweI, Mboll, Mlyl, Mmel, Mnll, Mva1269I, NmeAIII, PaqCI, PciSI, Pctl, Plel, Ppsl, Psrl, Schl, SfaNI, Taqll, TspDTI and/or TspGWI restriction enzyme.

The nuclease may be an exonuclease e.g. exonuclease I, exonuclease III and/or exonuclease VIII.

Each aspect or embodiment as defined herein may be combined with any other aspect(s) or embodiment(s) unless clearly indicated to the contrary. In particular any feature indicated as being preferred or advantageous may be combined with any other feature or features indicated as being preferred or advantageous.

The foregoing detailed description has been provided by way of explanation and illustration, and is not intended to limit the scope of the appended claims. Many variations in the presently preferred embodiments illustrated herein will be apparent to one of ordinary skill in the art, and remain within the scope of the appended claims and their equivalents.

**SEQUENCES**

| **SEQ ID NO.** | **Sequence** | **Description** |
|---|---|---|
| 1 | **N*ACAAATGTTAGCCCTN** | **F**irst adaptor first strand ligated |
| 2 | **NAGGGCTAACATTTGTN** | First adaptor second strand ligated |
| 3 | **NAGGGCTAACATTTGTN*** | First adaptor second strand ligated (with N*) |
| 4 | **NCATGCTAACATTTGN*** | Second adaptor first strand ligated |
| 5 | **NCAAATGTTAGCATGN** | Second adaptor second strand ligated |
| 6 | **N*CAAATGTTAGCATGN** | Second adaptor second strand ligated (with N*) |
| 7 | **N*ACAAATGTTAG** | First adaptor first strand |
| 8 | **AGGGCTAACATTTGTN** | First adaptor second strand |
| 9 | **AGGGCTAACATTTGTN*** | First adaptor second strand (with N*) |
| 10 | **CATGCTAACATTTGN*** | Second adaptor first strand |
| 11 | **NCAAATGTTAG** | Second adaptor second strand |
| 12 | **N*CAAATGTTAG** | Second adaptor second strand (with N*) |

### BRIEF DESCRIPTION OF THE DRAWINGS

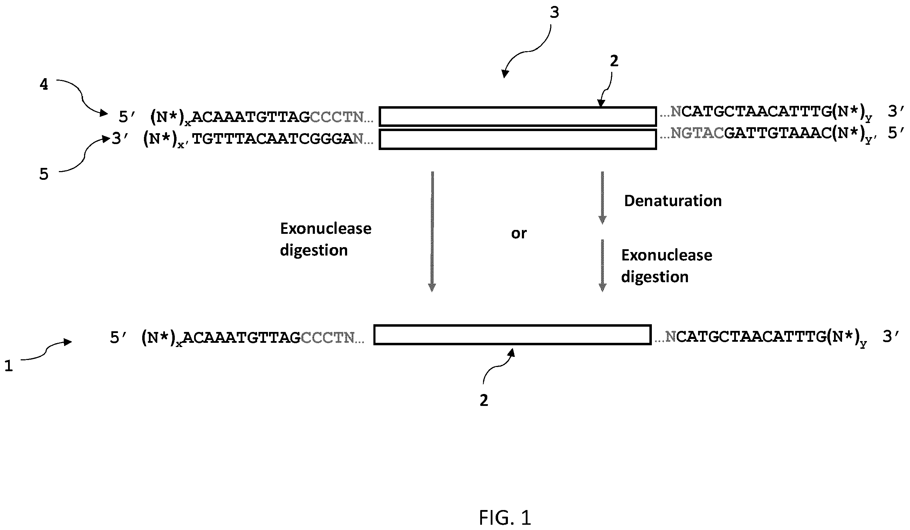

**FIG.1** shows a method for producing a protected DNA (1) comprising a single-stranded DNA (ssDNA) cassette (2), wherein the method comprises:
   (a) providing a partially protected double-stranded DNA (dsDNA) (3) comprising a first strand (4) and a second strand (5), wherein the first strand of the partially protected dsDNA comprises the cassette, x nuclease-resistant nucleotides (N*) at the 5' end of the cassette or 5' of the cassette, and y nuclease-resistant nucleotides at the 3'-end of the cassette or 3' of the cassette, wherein x is at least 1 and y is at least 1; and
   (b) digesting the second strand of the partially protected dsDNA with an exonuclease thereby generating the protected DNA.
   The second strand of the partially protected dsDNA may comprise x' and y' nuclease-resistant nucleotides (N*), wherein x' and y' may be 0.
   Digesting the second strand of the partially protected dsDNA may comprise contacting the partially protected dsDNA with an exonuclease. Alternatively, digesting the second strand of the partially protected dsDNA may comprise denaturing the partially protected dsDNA and contacting the second strand of the partially protected dsDNA with an exonuclease.

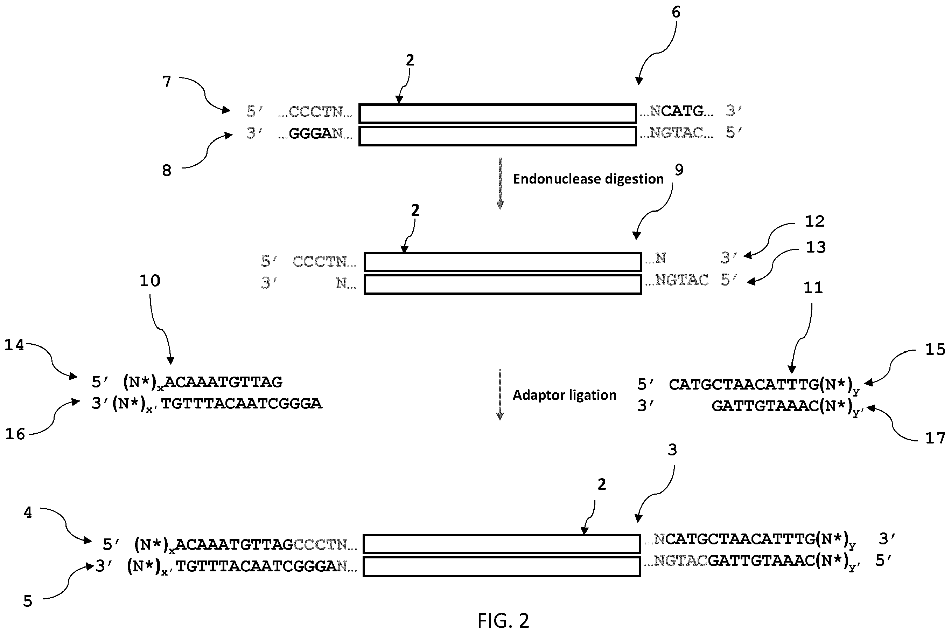

**FIG. 2** shows a method of generating the partially protected dsDNA (3) by:
   (a) contacting a precursor dsDNA (6) comprising a first (7) and a second (8) strand with a Bsal endonuclease, wherein the precursor dsDNA comprises on the first strand a cassette (2), an Bsal endonuclease target sequence 5' of the cassette and a Bsal endonuclease target sequence 3' of the cassette;
   (b) digesting the precursor dsDNA with the Bsal endonuclease to generate a digested precursor dsDNA (9);
   (c) contacting the digested precursor dsDNA with a ligase and first (10) and second adaptor molecules (11), wherein the first and second adaptor molecules each comprise a nuclease-resistant nucleotide;
   (d) ligating the first adaptor molecule a first end of the digested dsDNA and ligating the second adaptor molecule to a second end of the digested dsDNA thereby generating a partially protected dsDNA.
   The first and second adaptor molecules may comprise dsDNA comprising a first strand (14, 15) and a second strand (16, 17). The first strands of the first and second adaptor molecules may be ligated by the ligase to the first strand (12) of the digested precursor dsDNA molecule and the second strands of the first and second adaptor molecules may be ligated by the ligase to the second strand (13) of the digested precursor dsDNA molecule.
   Also shown are the sequences driving adaptor molecule ligation after Bsal digestion of a precursor dsDNA. Bsal digestion produces 4-nucleotide protruding ends at 5' (upstream TCCC 5' and downstream GTAC 5') at each side of the cassette. Upstream adaptors are formed by hybridization of complementary oligonucleotides (e.g. SEQ ID NO: 7 and 8 or 9) containing phosphorothioate internucleotide linkages (N*) in the 5' end and forming a 4-nucleotide protruding end at 5' (GGGA 5'). Downstream adaptors are formed by hybridization of complementary oligonucleotides (e.g. SEQ ID NO: 10 and 11 or 12) containing phosphorothioate internucleotide linkages (N*) in the 3' end and forming a 4-nucleotide protruding end at 5' (GTAC 5'). Complementary adaptor molecules are ligated at each side of the cassette, resulting in a partially protected dsDNA comprising protected nucleotides on both ends of one strand.

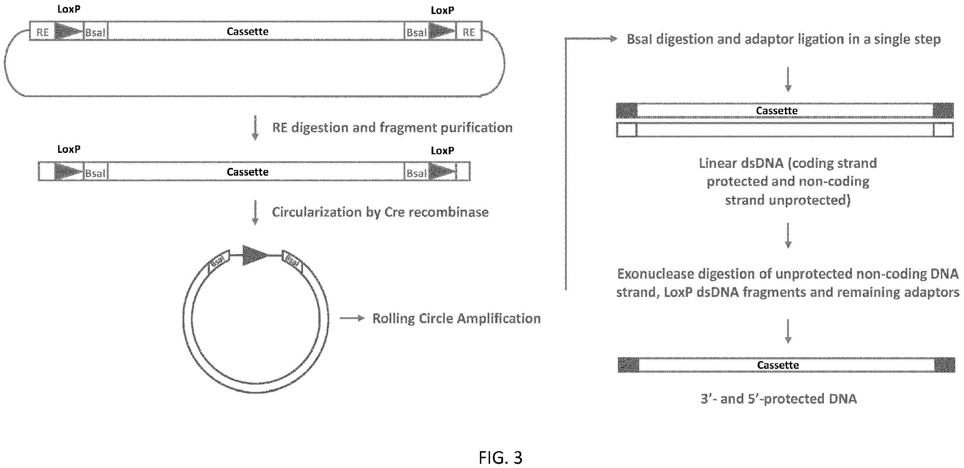

**FIG. 3** shows the generation of a circular DNA template obtained from a Cre recombinase reaction. Rolling circle amplification generates the precursor dsDNA which undergoes Bsal digestion and ligation with adaptor molecules to obtain the partially protected precursor dsDNA comprising a first strand (i.e. protected coding strand) and a second strand (i.e. unprotected non-coding strand). Exonuclease digestion of the second strand yields the protected DNA comprising a ssDNA cassette.

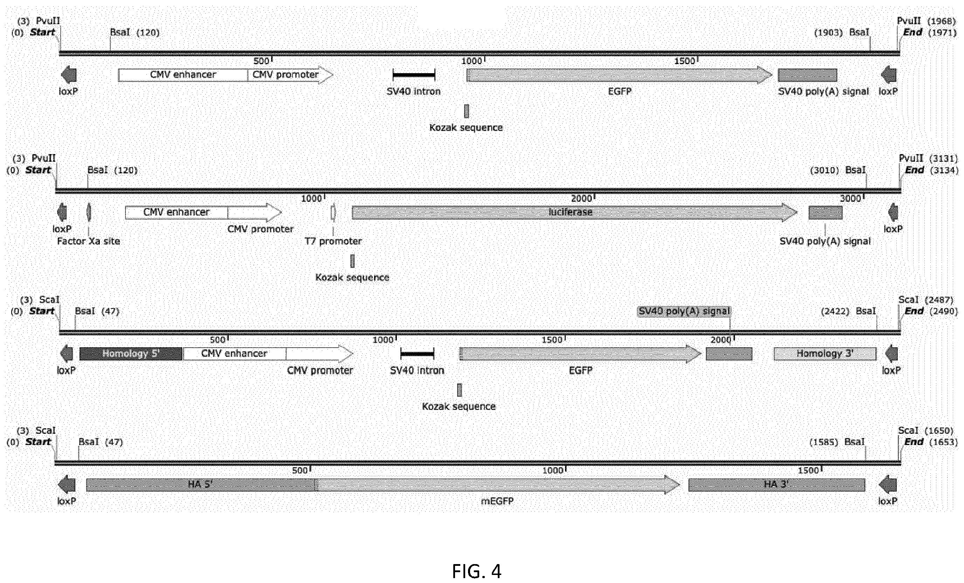

**FIG. 4** illustrates the elements forming four different expression cassettes suitable for the generation of single-stranded DNA comprising protected nucleotides on both ends.

### EXAMPLES

### Example 1

A plasmid containing any of the expression cassettes shown in FIG. 4 was subjected to the procedure depicted in FIG. 3.

Cre recombinase from the P1 bacteriophage is a Type I topoisomerase. The enzyme catalyzes the site-specific recombination of DNA between IoxP sites. LoxP recognition site (34 bp) consists of two 13 bp inverted repeats which flank an 8 bp spacer region, which confers directionality. The products of Cre-mediated recombination are dependent upon the location and relative orientation of the IoxP sites. Two DNA species containing single IoxP sites were fused. DNA found between two IoxP sites oriented in the same direction was excised as a circular loop of DNA, while DNA between opposing IoxP sites was inverted with respect to external sequences. Cre recombinase requires no additional cofactors or accessory proteins for its function.

**Cre reaction conditions:** reaction volume 1 ml, DNA of interest purified after restriction enzyme digestion (2 ng/µl), Cre recombinase (NEB, 0,08 units/µl), incubation time and temperature: 30 min at 37°C and 20 min at 80°C. Next, to remove remaining non-circular DNA molecules before the amplification step, E. *coli* exonuclease I (NEB, 0,4 units/µl) and III (NEB, 2 units/µl) were added and the reaction was incubated 30 min at 37°C and 20 min at 80°C.

Rolling circle amplification (RCA) is a faithful and isothermal DNA amplification method based on Phi29 DNA polymerase (Phi29DNApol). Phi29DNApol is the monomeric enzyme responsible for the replication of the linear double stranded DNA of bacteriophage phi29 from *Bacillus subtilis* (Blanco and Salas, 1984). It is an extremely processive polymerase (up to more than 70 kb per binding event) with a strong strand displacement capacity (Blanco *et al,* 1989). The enzyme displays 3'->5' proofreading exonuclease activity (Garmendia *et al,* 1992), resulting in an extremely high fidelity of synthesis (Esteban *et al,* 1993). These special features make this enzyme the perfect choice for isothermal DNA amplification.

RCA can be initiated by random synthetic primers (Dean *et al,* 2001) or a DNA primase like *Tth*PrimPol (Picher *et al,* 2016) that synthesizes the primers for Phi29DNApol during the amplification reaction.

**Rolling Circle Amplification (RCA):** before the amplification, circularized DNA was first denatured by adding 1 volume of buffer D (400 mM KOH, 10 mM EDTA) and incubating 3 min at room temperature. The sample was then neutralized by adding 1 volume of buffer N (400 mM HCI, 600 mM Tris-HCI pH 7.5). Rolling circle amplification conditions: 20 ml reaction volume, 2 ml TruePrime WGA reaction buffer 10x (4basebio), 3 ml denatured DNA sample, 2 ml TthPrimPol (1 µM), 320 µl QualiPhi Phi29DNApol (12,5 µM), 5 units PPase (Thermo) and 2 ml dNTPs (10 mM). Incubation time and temperature: 20 hours at 30°C and 10 min at 65°C.

**DNA digestion and adaptor ligation:** amplified DNA was then incubated with Type II restriction enzyme Bsal, T4 DNA ligase and complementary adaptors as defined herein to the 5' protruding ends generated by Bsal on the amplified DNA. Digestion and ligation reaction conditions: reaction volume 20 ml, 2 ml reaction buffer T4 DNA ligase 10x (NEB), 240 ng/µl amplified DNA, 0,6 units/µl Bsal-HFv2 (NEB), 20 units/µl T4 DNA ligase (NEB), DNA adaptors (1:10 molar excess), incubation time and temperature: 23 hours at 30°C.

### Exonuclease treatment to generate protected DNA comprising a ssDNA cassette:

1) Double-stranded exonuclease reaction conditions (i.e. using an exonuclease which acts on dsDNA): 0,75 units/µl of E. coli exonuclease III (NEB) were then added to degrade non-coding unprotected strands and coding strands lacking adaptors at both ends and remove remaining adaptors and LoxP fragments. Incubation time and temperature: 2 hours at 37°C.
2) Single-stranded exonuclease reaction conditions (i.e. using an exonuclease which acts on ssDNA): before exonuclease treatment, DNA was denatured by heat (3' at 95°C), directly cooling the denatured sample to 4°C on ice to prevent double-stranded formation. Alternatively, DNA was denatured by adding 1 volume of buffer D (400 mM KOH, 10 mM EDTA) and incubating 3 min at room temperature. The sample was then neutralized by adding 1 volume of buffer N (400 mM HCI, 600 mM Tris-HCI pH 7.5). 0,15 units/µl of E. coli exonuclease I (NEB) were then added to degrade non-coding unprotected strands and coding strands lacking adaptors at both ends and remove remaining adaptors and LoxP fragments. Incubation time and temperature: 2 hours at 37°C.
3) Single- and double-stranded exonuclease reaction conditions: 0,75 units/µl of E. coli exonuclease III (NEB) and 0,15 units/µl of E. coli exonuclease I (NEB) were simultaneously added to degrade non-coding unprotected strands and coding strands lacking adaptors at both ends and remove remaining adaptors and LoxP fragments. Incubation time and temperature: 2 hours at 37°C.

## Claims

1. A protected DNA comprising a single-stranded DNA (ssDNA) cassette, wherein the protected DNA comprises x nuclease-resistant nucleotides at the 5' end of the ssDNA cassette or 5' of the ssDNA cassette, and y nuclease-resistant nucleotides at the 3'-end of the ssDNA cassette or 3' of the ssDNA cassette, wherein x is at least 1 and y is at least 1 and wherein the ssDNA cassette comprises at least 100 nucleotides.

2. A partially protected double-stranded DNA (dsDNA) comprising a first strand and a second strand, wherein the first strand comprises:
(i) a cassette;
(ii) x nuclease-resistant nucleotides at the 5' end of the cassette or 5' of the cassette; and
(iii) y nuclease-resistant nucleotides at the 3'-end of the cassette or 3' of the cassette;
wherein x is at least 1 and y is at least 1 and wherein the second strand of the dsDNA molecule does not comprise any nuclease-resistant nucleotides.

3. A method for producing a protected DNA comprising a single-stranded DNA (ssDNA) cassette, wherein the method comprises:
(a) providing a partially protected double-stranded DNA (dsDNA) comprising a first strand and a second strand, wherein the first strand of the partially protected dsDNA comprises a cassette, x nuclease-resistant nucleotides at the 5' end of the cassette or 5' of the cassette, and y nuclease-resistant nucleotides at the 3'-end of the cassette or 3' of the cassette, wherein x is at least 1 and y is at least 1; and
(b) digesting the second strand of the partially protected dsDNA with an exonuclease thereby generating the protected DNA.

4. The method of claim 3, wherein the partially protected dsDNA is generated by:
(a) contacting a precursor dsDNA comprising a first strand and a second strand with an endonuclease, wherein the precursor dsDNA comprises on the first strand a cassette, an endonuclease target sequence 5' of the cassette and an endonuclease target sequence 3' of the cassette;
(b) digesting the precursor dsDNA with the endonuclease to generate a digested precursor dsDNA;
(c) contacting the digested precursor dsDNA with a ligase and first and second adaptor molecules, wherein the first adaptor molecule comprises at least x nuclease resistant nucleotides and the second adaptor molecule comprises at least y nuclease-resistant nucleotides and wherein x is at least 1 and y is at least 1; and
(d) ligating the first adaptor molecule to a first end of the digested precursor dsDNA and ligating the second adaptor molecule to a second end of the digested precursor dsDNA thereby generating the partially protected dsDNA.

5. The method of claim 4, wherein the method further comprises:
amplifying a DNA template to generate the precursor dsDNA, wherein the DNA template comprises the cassette and the endonuclease target sequences, optionally wherein the DNA template is amplified by rolling circle amplification.

6. The method of claim 4 or claim 5, wherein the first and second adaptor molecules comprise dsDNA comprising a first strand and a second strand, and wherein the first strands of the first and second adaptor molecules are ligated by the ligase to the first strand of the digested precursor dsDNA molecule and the second strands of the first and second adaptor molecules are ligated by the ligase to the second strand of the digested precursor dsDNA molecule.

7. The method of any one of claims 4 to 6, wherein the first adaptor molecule comprises an overhang complementary to an overhang at the first end of the digested precursor dsDNA and the second adaptor molecule comprises an overhang complementary to an overhang at the second end of the digested precursor dsDNA.

8. The protected DNA of claim 1, the partially protected dsDNA of claim 2 or the method of any one of claims 3 to 7, wherein the cassette comprises at least 100 nucleotides, at least 200 nucleotides, 500 nucleotides, 1000 nucleotides, 5000 nucleotides or 10000 nucleotides.

9. A kit comprising:
(a) first and second adaptor molecules, wherein each first and second adaptor molecule comprises dsDNA comprising a first strand and a second strand, wherein the first strand of the first adaptor molecule comprises x nuclease-resistant nucleotides and the first strand of the second adaptor molecule comprises y nuclease-resistant nucleotides, wherein x is at least 1 and y is at least 1;
(b) an endonuclease;
(c) a ligase; and
(d) an exonuclease.

10. The method of any one of claims 4 to 8 or the kit of claim 9, wherein the endonuclease is a Type IIS restriction endonuclease.

11. The protected DNA of claim 1, the partially protected dsDNA of claim 2, the method of any one of claims 3 to 8 and 10 or the kit of claim 9 or 10, wherein x is at least 3 and y is at least 3, optionally wherein x is at least 5 and y is at least 5.

12. A method for producing a protein, wherein the method comprises:
(a) providing a protected DNA as defined in any one of claims 1, 8 and 11, or producing a protected DNA according to the method of any one of claims 3 to 8, 10 and 11;
(b) introducing the protected DNA into a cell or a cell-free expression system to generate a protein encoded by the protected DNA .

13. A method for cell transfection of a single-stranded deoxyribonucleic acid (ssDNA) product into a cell, wherein the method comprises:
(a) providing a protected DNA as defined in any one of claims 1, 8 and 11, or producing a protected DNA according to the method of any one of claims 3 to 8, 10 and 11;
(b) contacting a cell with the protected DNA; and
(c) transfecting the protected DNA into the cytosol of the cell.

14. Use of a protected DNA in the production of viral or non-viral delivery system, wherein the protected DNA is as defined in any one of claims 1, 8 and 11, or where the protected DNA is produced by performing the method of any one of claims 3 to 8, 10 and 11.

15. Use of a protected DNA in gene editing, wherein the protected DNA is as defined in any one of claims 1, 8 and 11, or where the protected DNA is produced by the method of any one of claims 3 to 8, 10 and 11, optionally wherein the gene editing uses a CRISPR associated (Cas) nuclease, a Transcription activator-like effector nuclease (TALEN) and/or a Zinc finger nuclease (ZFN).
